# EUROPEAN PATENT SPECIFICATION

(11) **EP 3 490 514 B1**
(45) Date of publication and mention of the grant of the patent: **06.01.2021**
(21) Application number: 17757839.0
(22) Date of filing: 17.08.2017
(51) Int. Cl.: A61F 13/00, A61L 15/00, C12Q 1/37

(54) **DETERMINING THE CONDITION OF A WOUND**
BESTIMMUNG DES ZUSTANDS EINER WUNDE
DÉTERMINATION D'ÉTAT D'UNE BLESSURE

(30) Priority: 17.08.2016 GB 201614053
(43) Date of publication of application: 05.06.2019
(73) Proprietor: Microarray Limited, Bedford, Bedfordshire MK44 1LQ (GB)
(72) Inventor: AUSTIN, Andrew, Bedford Bedfordshire MK44 1LQ (GB); LEADBEATER, Paul, Bedford Bedfordshire MK44 1LQ (GB); DOBB, Roy, Bedford Bedfordshire MK44 1LQ (GB)
(74) Representative: Boult Wade Tennant LLP
(86) International application number: PCT/GB2017/052435
(87) International publication number: WO 2018/033739

(56) References cited:
- WO-A1-2012/171922
- WO-A1-2016/128762
- US-A- 5 466 567
- US-A- 5 744 545
- US-A1- 2002 121 208
- US-A1- 2016 069 884
- US-A1- 2016 069 905

## Description

### FIELD OF THE INVENTION

The invention generally relates to products and associated methods for determining the condition of a wound, which may be a chronic wound.

### BACKGROUND TO THE INVENTION

A wound may be defined as a breakdown in the protective function of the skin; the loss of continuity of epithelium, with or without loss of underlying connective tissue (i.e. muscle, bone, nerves) following injury to the skin or underlying tissues/ organs caused, for example, by surgery, a blow, a cut, chemicals, heat/ cold, friction/ shear force, pressure or as a result of disease, such as leg ulcers or carcinomas (Leaper and Harding, Wounds: Biology and Management, Oxford University Press (1998)).

Wound healing comprises restoration of any damaged tissue comprising formation of new connective tissues and re-growth of epithelium (Copper, A review of different wound types and their principles of management in Wound Healing: A systematic approach to advanced wound healing and management, Cromwell Press, UK (2005)).

Wounds can be classified as acute or chronic. Acute wounds comprise those in which healing occurs as a sequential cascade of overlapping processes that requires the coordinated completion of a variety of cellular activities. Conversely, a chronic wound is one in which the normal process of wound healing is disrupted at one or more points in the phases of wound healing. Often this may lead to a chronic wound becoming stuck in a particular phase of healing such as inflammation or proliferation. Chronic wounds are often identified by the presence of a raised, hyperproliferative, yet nonadvancing wound edge. The local wound environment, rich in inflammatory products, and proinflammatory cytokines may comprise an imbalanced enzymatic milieu consisting of an excess of matrix metalloproteases and a reduction in their inhibitors resulting in the destruction of the extracellular matrix (Menke et al., Impaired wound healing, Clinical Dermatology (2007)). The resultant profound inflammatory state is thought to be a significant factor influencing and delaying healing. Furthermore, chronic wounds can often become impeded by the accumulation of necrotic or sloughy tissue in the wound bed. It has been reported that, in the US alone, chronic wounds affect approximately 5.7 million patients and cost an estimated US$20 billion annually (Branski et al., A review of gene and stem cell therapy in cutaneous wound healing, Burns (2008)). Common chronic wounds include diabetic ulcers, vascular ulcers and pressure ulcers (Werdin et al., Evidence-based Management Strategies for Treatment of Chronic Wounds, Eplasty (2009)).

Management of wound healing has been suggested to comprise four principal elements: the tissue within and surrounding the wound and its status, the presence of any inflammation and/or infection within or surrounding the wound, the moisture balance within the wound and the quality of the wound edge (Ayello et al., TIME heals all wounds, Nursing (2004)).

At present, wound dressings are available which seek to address one or more of these principal elements. Choosing an appropriate wound dressing comprises consideration of the current phase of wound healing, its specific temporal requirements, as well as potential side effects. Ideally, dressings should minimize pain and be easy to use. These dressings must prevent friction and shear while protecting the peri-ulcer tissue and skin. A combination of different dressings at different stages of the healing process has been proposed. For instance, the use of hydrogel dressings for the debridement phase, foam dressings at the granulation stage, and the use of either hydrocolloids or low adherence dressings for the epithelialization phase (Vaneau et al., Consensus panel recommendations for chronic and acute wound dressings, Archives of Dermatology (2007)).

US 5,181,905 relates to a dressing for a wound on or within which is an indicator which conveys information as to the condition of the wound. The exemplified embodiments and preferred indicator comprises temperature sensitive liquid crystals which can change colour at specific temperatures.

US 2004/0044299 A1 relates to a wound dressing comprising on or within it a chemical composition that changes colour in the presence of bacteria and which is visible to the naked eye through the outer surface of the dressing.

GB 2340235 A relates to a method of monitoring bacterial contamination of a wound based on the concentration of ATP in the wound exudate, along with companion devices and kits. WO 2012/074509 relates to patch-based sensors that provide a panel of specific analyte parameters that determine one or more physiological conditions and/or the level of healing progression of a wound.

US 2009/203059 A1 relates to a protease detection product for detecting a protease enzyme in a sample.

US 2014/227773 A1 relates to methods and devices for detecting a target enzyme comprising an anchored or trapped peptide complex.

### SUMMARY OF THE INVENTION

Monitoring of the condition of the wound is currently limited to an initial assessment by the caregiver responsible for routine changing of the wound dressing comprising evaluation of one or more of the appearance and/or smell of the wound and/or the volume of exudate production. Such an assessment may suggest to the caregiver that referral of the patient to a clinical practitioner or further analysis of the wound and/or wound exudate is required. However, such assessments are unable to analyse one or more components of the exudate at the point of care to indicate the condition of the wound.

In view of this, International patent application WO2016/128762, published on 18 August 2016, relates to a product for monitoring the condition of a wound comprising:
(i) a biologically inert matrix which absorbs wound exudate; and
(ii) one or more reagents on or in the matrix for measuring one or more markers comprised within the wound exudate
wherein a change in the one or more reagents caused by the one or more markers comprised within the wound exudate provides a visual indication of an alteration in the condition of the wound.

The present invention relates to developments of this technology. The first development is in relation to the use of cross-linked and protease-sensitive polymers that are degraded only when protease activity in the wound exudate is (sufficiently) present, preferably at or above a pre-determined threshold level. The second development relates to the use of protease-sensitive polymers in a lateral flow-based device for *ex situ* monitoring of the condition of the wound. Consequently, the products described herein provide simple, easy-to-use easy-to-interpret and low cost means to monitor the condition of a wound.

Thus, as defined in the claims, the invention provides a product for monitoring the condition of a wound comprising a matrix that absorbs wound fluid, the matrix comprising:
a. cross-linked and protease-sensitive polymers forming a reaction zone on/in the matrix; and
b. coloured particles;
wherein the arrangement of the polymers and coloured particles is such that cleavage of the polymers by protease activity present in the wound fluid results in transport of the coloured particles along/through the matrix to provide a visual indication of protease activity in the wound fluid;
wherein the matrix comprises a viewing zone that, prior to exposure to wound fluid and in the absence of protease activity, does not contain coloured particles and wherein cleavage of the polymers by protease activity present in the wound fluid results in release of the coloured particles along/through the matrix providing a visual indication of protease activity in the viewing zone, wherein:
(i) the coloured particles are entrapped within the polymers and cleavage of the polymers by protease activity present in the wound fluid results in release of the coloured particles along/through the matrix to provide a visual indication of protease activity in the wound fluid; or
(ii) the cross-linked and protease-sensitive polymers form a barrier that, in the absence of protease activity, optionally above a threshold level, in the wound fluid, prevents the wound fluid from coming into contact with the coloured particles and wherein cleavage of the polymers by protease activity present in the wound fluid, optionally above a threshold level, disrupts the barrier and results in flow of the coloured particles along/through the matrix to provide a visual indication of protease activity in the wound fluid in the viewing zone.

Central to the invention is the fact that the cross-linked and protease-sensitive polymers and coloured particles are arranged (relative to each other) such that, when the matrix is contacted with and absorbs a (sufficient) amount of wound fluid, cleavage of the polymers by protease activity present in the wound fluid results in (permits) transport of the coloured particles along/through the matrix to provide a visual indication of protease activity in the wound fluid. Typically, this transport of coloured particles is away from the reaction zone. It may be towards and into a viewing zone as defined and discussed herein. Furthermore, as further described herein, the cross-linking of the polymers enhances the resistance of the polymers to cleavage by protease activity (compared to a non-cross-linked version). Thus, unless and until protease activity is present in sufficient amounts to indicate a change in condition of the wound, which may be at or above a (pre-determined) threshold level of protease activity, the coloured particles remain fixed in (location in) the matrix. The threshold level of activity is indicative of a non-healing wound. Average and median levels of protease activity in acute wounds versus chronic (non-healing) wounds, along with methods for deriving such levels, are known in the art (see Trengove et al., Wound Rep. Reg., 1999, vol. 7, pages 442-452). Thus, appropriate threshold levels can be derived for general and/or personalised use. Forming a reaction zone of suitable protease sensitivity can be determined and adjusted empirically, for instance, based on the time the product is due to be left in contact with wound fluid (e.g. under a wound dressing). Protease sensitivity can be controlled for example by adjusting the degree of cross-linking between polymer molecules as described herein.

Transmission of wound fluid along/through the matrix (along with coloured particles should protease activity be present in the wound fluid) may be by any suitable means. Typically, wound fluid moves along/through the matrix by capillary action.

"Wound" can be defined as a breakdown in the protective function of the skin; the loss of continuity of epithelium, with or without loss of underlying connective tissue (i.e. muscle, bone, nerves) following injury to the skin or underlying tissues/ organs caused, for example, by surgery, a blow, a cut, chemicals, heat/ cold, friction/ shear force, pressure or as a result of disease, such as leg ulcers or carcinomas.

"Wound fluid", also termed "wound exudate", should be understood to mean the fluid environment of the wound which is exposed to the external environment by virtue of the breakdown in the protective function of the skin and loss of continuity of epithelium comprising, consisting essentially of or consisting of pus, serum, water and/or blood and further comprising, consisting essentially of or consisting of one or more lipids, polysaccharides, proteins, in particular proteases such as extracellular matrix proteins including collagenases (more specifically, for example, gelatinases), and cellular debris.

The matrix absorbs wound fluid and thereby exposes the reaction zone to the wound fluid. In many embodiments, the reaction zone is formed on the top surface of the matrix and wound fluid is absorbed via the bottom surface. Thus, in such embodiments, the matrix permits the wound fluid to pass to the reaction zone. In some embodiments, the matrix is dimensioned such that the wound fluid saturates the matrix. The matrix may, in some embodiments, be biologically inert i.e. it does not interact with or initiate a response from biological tissue with which it comes into contact. Products comprising a biologically inert matrix are preferred for *in situ* applications of the products defined herein wherein the product is placed in contact with a wound for a pre-determined period of time. By being biologically inert the risk of an adverse immune reaction by the wounded subject is minimized and preferably negated altogether. It will be appreciated that the specific dimensions of a wound are unique in each case and that wound dressing size is adapted accordingly, for example using cut to size dressings. Consequently, in further embodiments for *in situ* use in a wound, the matrix has dimensions suitable for and intended to facilitate positioning of the product between a wound dressing and the wound. In certain embodiments, the matrix has thickness x width x length dimensions of at least 2mm x 10mm x 10mm but not more than 7mm x 40mm x 40mm. In a particular embodiment, the matrix has the dimensions 5mm x 25mm x 25mm. The top and bottom surfaces do not necessarily have to be square in all embodiments. They could be rectangular or circular for example. The matrix may be cylindrical in some embodiments. The matrix may also be provided in a cut to size format, provided each matrix once cut forms a product of the invention (i.e. produces a reaction zone and coloured particles suitably arranged in the matrix). For *in situ* application, the matrix is sufficiently soft and comfortable to minimise or avoid causing significant discomfort in the wound, particularly after the wound dressing has been applied. Application of the wound dressing exerts a level of compression on the matrix. Accordingly, in further embodiments, the matrix is sufficiently resistant to compression to allow the matrix to maintain a structure suitable to absorb sufficient volumes of wound fluid to allow the product to function. In particular embodiments, the matrix is able to absorb and retain a volume of wound fluid (exudate) sufficient for further (downstream and separate) analysis of the exudate as further described herein. For instance, in certain embodiments, the matrix has the capacity to absorb a volume of at least 0.2ml (wound fluid). In further embodiments, the matrix has the capacity to absorb a volume in the range of 0.2ml to 10ml. For instance, a volume of at least 0.2ml, 0.3ml, 0.4ml, 0.5ml, 1ml, 2ml, 3ml, 4ml, 5ml or 10ml. In a particular embodiment, the matrix has the capacity to absorb a volume of 3ml (to include a range of 2.5 to 3.4ml).

In certain embodiments, the matrix is composed of one or more materials selected from:
(i) polyurethane; and/or
(ii) polyethylene; and/or
(iii) cellulose fibres; and/or
(iv) porous hydrophilic plastic.

Suitable porous hydrophilic plastics include those marketed by Porex Limited.

In other embodiments, the matrix comprises a non-woven material such as Orion non-woven material available from Anowo Ltd. In specific embodiments, the non-woven material has a 4 osy weight.

Where the matrix performs dual functions (i.e. provides a visual indicator of protease activity in the wound fluid and also retains a volume of exudate sufficient for further analysis) the matrix may comprise, consist essentially of or consist of a first and second portion. Accordingly, throughout the disclosure, reference to "matrix" encompasses reference to first and/or second portions of a matrix. The matrix may thus comprise, consist essentially of or consist of first and second layers. These two portions or layers may be attached to each other, for instance, by lamination. Each portion has one or more or all of the features of the matrix as described herein.

In some embodiments, the first portion comprises, consists essentially of or consists of the cross-linked and protease-sensitive polymers forming a reaction zone thereon/therein. The first portion is able to absorb sufficient wound fluid to allow the cross-linked and protease-sensitive polymers (on or in the first portion) to come into contact with the wound fluid and, therefore, the protease activity that may be contained therein. The first portion also typically comprises, consists essentially of or consists of the coloured particles. Generally, the first portion is also the site of visualisation of protease activity in the wound fluid.
In some embodiments, the second portion is able to absorb wound fluid in an amount sufficient for downstream (and separate) analysis of the wound fluid as described further herein.

Accordingly, described herein is a product for monitoring the condition of a wound comprising, consisting essentially of or consisting of:
(i) a first (biologically inert) matrix that absorbs wound fluid which comprises, consists essentially of or consists of:
   a. cross-linked and protease-sensitive polymers forming a reaction zone on/in the matrix; and
   b. coloured particles;
   wherein the arrangement of the polymers and coloured particles is such that cleavage of the polymers by protease activity present in the wound fluid results in transport of the coloured particles along/through the matrix to provide a visual indication of protease activity in the wound fluid; and
(ii) a second (biologically inert) matrix which absorbs wound fluid in an amount sufficient for downstream (and separate) analysis of the wound fluid.

The matrix may thus be arranged such that the second portion comes directly into contact with the wound and the first portion indirectly absorbs wound fluid through fluid communication with the second portion. The first portion may thus be stacked on top of the second portion. The pressure applied by a wound dressing may keep the portions in fluid connect *in situ.* In other embodiments they may be more permanently connected, such as by lamination.

In some embodiments, the reaction zone may be effectively sandwiched between the first and second portions of the matrix, optionally together with the coloured particles (e.g. entrapped within the reaction zone). If there is sufficient protease activity in the wound fluid, the protease-sensitive polymers are degraded thereby releasing the coloured particles into the wound fluid. As the wound fluid passes into the first portion, the colour is visible on the upper surface.

In further embodiments, the surface of the first portion that is not in contact with the second portion is coated with or otherwise surrounded by a transparent film in order to protect it from physical damage. Wound fluid absorbed by the second portion may still access the first portion via connecting surface that permits fluid communication. Such an arrangement ensures that the cross-linked and protease-sensitive polymers comprised, consisting essentially of or consisting of on or in the first portion of the matrix are exposed to the wound fluid. Transparency of the film allows any signal generated as a consequence of the cross-linked and protease-sensitive polymers coming into contact with protease activity in the wound fluid, as further described herein, to be detected visually at the point of care. More generally, the top surface of the matrix may comprise, consist essentially of or consist of these features.

In some embodiments, the first portion or layer of the matrix is substantially thinner than the second portion. In some embodiments, the first portion or layer comprises, consists essentially of or consists of a membrane. In some embodiments, the second portion comprises, consists essentially of or consists of an absorbent foam material, such as polyurethane foam. In particular embodiments where the matrix is comprised, consists essentially of or consists of a first and second portion as described herein, the first and second portion may be composed of the same or different materials. In some embodiments, the second portion is composed of polyurethane, which may be in the form of a foam. In particular embodiments, the polyurethane is a non-isocyanate based polyurethane. In other embodiments, the first and/or second portion may comprise a non-woven material such as Orion non-woven material available from Anowo Ltd. In specific embodiments, the non-woven material has a 4 osy weight.

In particular embodiments, the coloured particles are entrapped within the polymers. Thus, the reaction zone in some embodiments comprises, consists essentially of or consists of the coloured particles. In such embodiments, the coloured particles are not released unless there is (sufficient) protease activity in the sample. Thus, exposure to wound fluid containing insufficient (including no) protease activity does not result in release of the coloured particles from the reaction zone (to any appreciable degree). Cleavage of the polymers by protease activity present in the wound fluid results in release of the coloured particles from the polymers along/through the matrix to provide a visual indication of protease activity in the wound fluid.

In some embodiments, the visual indication of protease activity in the wound fluid which is provided following release of the coloured particles along/through the matrix after cleavage of the polymers by protease activity present in the wound fluid comprises, consists essentially of or consists of dispersal of the coloured particles from their initial zone of deposition on the matrix (i.e. from the reaction zone, as defined prior to contact with the wound fluid). Thus, colouration in the reaction zone reduces in intensity or disappears altogether. The reduction in intensity is visually perceptible. In some embodiments, a colour guide may be provided with the product to provide a reference for the expected colour changes/levels if the appropriate proteases are active in the wound fluid. This may be a scale, for example a simple scale of no, low or high activity, or a numerical scale in some embodiments. The scale may be provided with recommendations for further action depending on the outcome of the reading. Alternatively, or in addition to, observance via the naked eye, the reduction in colour intensity can be detected and/or quantified using a spectrophotometer or other such equipment suitable for this purpose, which are well-known to the skilled person. Thus, the products of the invention may be provided with a suitable reader or may be integrated into an apparatus capable of performing some level of automated reading of the signal. This may be quantitative, or semi-quantitative in some embodiments.

In particular embodiments, the matrix comprises a visual symbol that, prior to exposure to wound fluid and in the absence of protease activity, is masked by the coloured particles, which may be entrapped within the polymers. For instance, the visual symbol may be printed on the matrix using any suitable means (e.g. indelible ink) over which the coloured particles (which may be entrapped within the polymers) are initially deposited. Once the matrix is contacted with and absorbs a (sufficient) amount of wound fluid, cleavage of the polymers by protease activity present in the wound fluid results in transport of the coloured particles along/through the matrix thereby revealing the visual symbol.

As defined in the claims, the matrix comprises a viewing zone. Prior to exposure to wound fluid and in the absence of protease activity, the viewing zone does not contain coloured particles. The viewing zone is thus separated from the reaction zone. Typically the separation is in at least two dimensions on the surface of the matrix. Upon exposure to wound fluid, cleavage of the polymers by protease activity present in the wound fluid results in release of the coloured particles along/through the matrix into the viewing zone. As a consequence, the viewing zone appears to change and/or intensify in colour by virtue of the movement of the coloured particles into the viewing zone. Hence, a visual indication of protease activity is provided in the viewing zone. In preferred embodiments, the change and/or intensification in colour is visible to the naked eye. Alternatively, or in addition to, observance via the naked eye, the change and/or intensification in colour can be detected and/or quantified using a spectrophotometer or other such equipment suitable for this purpose, which are well-known to the skilled person. The matrix may be arranged such that wound fluid flow is preferably in the direction of the viewing zone from the reaction zone. Thus, for example, the matrix may comprise a channel directing fluid flow from the reaction zone to the viewing zone in some embodiments. This is not necessary in all embodiments, however. In some embodiments, the viewing zone may simply be the visible surface of the matrix that is not the reaction zone and/or the location of the coloured particles in the absence of (sufficient) protease activity.

In particular embodiments, the viewing zone comprises, consists essentially of or consists of capture molecules to capture coloured particles. This may assist with generation of an easily interpreted signal. The capture molecules may be arranged to produce a visible line or other symbol that may permit some level of quantitation of the signal (and thus of protease activity). The capture molecules are any molecules capable of binding to the coloured particles. These may comprise, consist essentially of or consist of, for instance, an antibody or aptamer that binds specifically to the coloured particles. In specific embodiments, the capture molecules are antibodies that bind specifically to the coloured particles. The antibodies may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Methods of generating specific antibodies and aptamers are well-known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In further embodiments, the matrix comprises a barrier aligned with the (downstream) end of the viewing zone. That is to say that the viewing zone is positioned between the coloured particles and the barrier and the barrier is positioned relative to the viewing zone such that coloured particles accumulate in the viewing zone following the release of the coloured particles along/through the matrix into the viewing zone after cleavage of the polymers by protease activity present in the wound fluid. Thus, the barrier prevents released coloured particles from travelling beyond the viewing zone. For instance, the barrier may comprise, consist essentially of or consist of a porous material wherein the pores are of a size that allows fluid and molecules with a molecular weight below a cut-off value to pass through the barrier but which are not large enough to allow the coloured particles to travel past the barrier. The skilled person is well able to select suitable materials with an appropriate molecular weight cut-off dependent on the coloured particles employed. Alternatively, for embodiments comprising a porous matrix, the barrier may comprise a region of the matrix itself wherein the region comprises pores with a pore size smaller than the diameter of the coloured particles (whilst still allowing the passage of fluid). Such a region may be created, for instance, by crushing or otherwise compressing this region of the matrix. The compression is to a degree sufficient to decrease the pore size such that it is smaller than the diameter of the coloured particles. Thus, the coloured particles cannot pass through the crushed/compressed region (barrier) and therefore accumulate in the viewing zone following the release of the coloured particles along/through the matrix into the viewing zone after cleavage of the polymers by protease activity present in the wound fluid.

According to all aspects and embodiments of the invention described herein, the coloured particles may comprise, consist essentially of or consist of coloured microparticles. Typically the coloured particles are insoluble (i.e. do not dissolve) in the wound fluid. In particular embodiments, the coloured particles may comprise, consist essentially of or consist of activated carbon particles and/or coloured polystyrene microparticles (such as Polybead® Polystyrene Blur Dyed Microsphere (0.5 micron diameter, 2.5% solids) sourced from Polysciences, Inc. (product number 15709)). In preferred embodiments, the coloured particles comprise, consist essentially of or consist of coloured polystyrene microparticles. Other non-limiting embodiments of coloured particles for use in the invention include copper phthalocyanine tetrasulfonic acid tetrasodium salt, coloured latex microparticles, gold particles and/or dye molecules such as phthalocyanine Blue BN (also called "Monastal Blue"; CAS 147-14-8). In general, the coloured particles are any coloured particles that can be detected as described herein or using methods known to those skilled in the art and which are able to be carried by an aqueous fluid along/through the matrix.

According to all aspects and embodiments of the invention described herein, entrapment of the coloured particles within the (optionally cross-linked) protease-sensitive polymers may be achieved by drying the polymers with the coloured particles. As a consequence, the polymers, and thus the reaction zone, adopt the colouration of the entrapped particles. The coloured particles remain entrapped within the polymers until and unless the polymers are cleaved by protease activity present in the wound fluid absorbed by the product. Following cleavage of the polymers by protease activity present in the wound fluid, the coloured particles are transported along/through the matrix to provide a visual indication of protease activity in the wound fluid. Typically, this is from the reaction zone to the viewing zone. Entrapment of coloured particles can be directly contrasted with use of soluble dyes that are associated with the protease-sensitive polymers. The association may be direct (e.g. covalent association) or indirect (e.g. adsorption). The coloured particles are advantageous in that they provide improved colouration of the reaction zone.

It will be apparent to the skilled person, in view of the foregoing, that particular embodiments of the invention described above lend themselves to application in an *ex situ* lateral flow-based format. Thus, the disclosure also provides:
A product for monitoring the condition of a wound comprising, consisting essentially of or consisting of:
a. a sample application zone to which wound fluid is added
b. a reaction zone downstream of the sample application zone comprising, consisting essentially of or consisting of cross-linked protease-sensitive polymers wherein coloured particles are entrapped within the polymers; and optionally
c. a viewing zone downstream of the sample application zone and reaction zone;
wherein the sample application zone transmits fluid towards the reaction zone (and viewing zone if present) and wherein cleavage of the polymers by protease activity present in the wound fluid, optionally above a threshold level, results in carriage of the coloured particles with the wound fluid along/through the matrix thereby providing a visual indication of protease activity (which can be observed in the viewing zone if present). The embodiments described above may apply to this specific lateral flow aspect *mutatis mutandis* and are not repeated here simply for conciseness.

In further embodiments, according to all foregoing aspects and embodiments, the cross-linked and protease-sensitive polymers (i.e. the reaction zone) form a barrier that, in the absence of protease activity, optionally above a threshold level, in the wound fluid, prevents the wound fluid from coming into contact with the coloured particles. Thus, the reaction zone may form a barrier to flow of wound fluid, unless there is sufficient protease activity in the wound fluid to break down the barrier. In specific embodiments, the coloured particles are not necessarily entrapped within the polymers but are downstream of the barrier formed by the polymers, that is to say the barrier is positioned between the point of application of the wound fluid on the matrix and the zone on the matrix where the coloured particles have been initially deposited. In use, cleavage of the polymers by protease activity present in the wound fluid, optionally above a threshold level, disrupts the barrier and results in flow of the coloured particles along/through the matrix to provide a visual indication of protease activity in the wound fluid. The matrix further comprises a viewing zone that, prior to exposure to wound fluid and in the absence of protease activity, does not contain coloured particles. Upon exposure to wound fluid, cleavage of the polymers by protease activity present in the wound fluid results in release of the coloured particles along/through the matrix into the viewing zone. As a consequence, the viewing zone appears to change and/or intensify in colour by virtue of the movement of the coloured particles into the viewing zone. Hence, a visual indication of protease activity is provided in the viewing zone.

In preferred embodiments, the change and/or intensification in colour is visible to the naked eye. Alternatively, or in addition to, observance via the naked eye, the change and/or intensification in colour can be detected and/or quantified using a spectrophotometer or other such equipment suitable for this purpose, which are well-known to the skilled person. Thus, the products of the invention may be provided with a suitable reader or may be integrated into an apparatus capable of performing some level of automated reading of the signal. This may be quantitative, or semi-quantitative in some embodiments.

In particular embodiments, the viewing zone comprises, consists essentially of or consists of capture molecules to capture coloured particles. This may assist with generation of an easily interpreted signal. The capture molecules may be arranged to produce a visible line or other symbol that may permit some level of quantitation of the signal (and thus of protease activity). The capture molecules are any molecules capable of binding to the coloured particles. These may comprise, consist essentially of or consist of, for instance, an antibody or aptamer that binds specifically to the coloured particles. In specific embodiments, the capture molecules are antibodies that bind specifically to the coloured particles. The antibodies may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Methods of generating specific antibodies and aptamers are well-known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In further embodiments, the matrix comprises a second barrier (i.e. in addition to the barrier formed by the cross-linked, protease-sensitive polymers) aligned with the (downstream) end of the viewing zone. That is to say that the viewing zone is positioned between the coloured particles and the second barrier and the second barrier is positioned relative to the viewing zone such that coloured particles accumulate in the viewing zone following the release of the coloured particles along/through the matrix into the viewing zone after cleavage of the polymers by protease activity present in the wound fluid. Thus, the second barrier prevents released coloured particles from travelling beyond the viewing zone. For instance, the barrier may comprise, consist essentially of or consist of a porous material wherein the pores are of a size that allows fluid and molecules with a molecular weight below a cut-off value to pass through the barrier but which are not large enough to allow the coloured particles to travel past the barrier. The skilled person is well able to select suitable materials with an appropriate molecular weight cut-off dependent on the coloured particles employed. Alternatively, for embodiments comprising a porous matrix, the second barrier may comprise a region of the matrix itself wherein the region comprises pores with a pore size smaller than the diameter of the coloured particles (whilst still allowing the passage of fluid). Such a region may be created, for instance, by crushing or otherwise compressing this region of the matrix. The compression is to a degree sufficient to decrease the pore size such that it is smaller than the diameter of the coloured particles. Thus, the coloured particles cannot pass through the crushed/compressed region (second barrier) and therefore accumulate in the viewing zone following the release of the coloured particles along/through the matrix into the viewing zone after cleavage of the polymers by protease activity present in the wound fluid.

In particular embodiments, the matrix comprises a visual symbol that, prior to exposure to wound fluid and in the absence of protease activity, is masked by the coloured particles. For instance, the visual symbol may be printed on the matrix using any suitable means (e.g. indelible ink) over which the coloured particles are initially deposited. Once the matrix is contacted with and absorbs a (sufficient) amount of wound fluid, cleavage of the polymers by protease activity present in the wound fluid disrupts the barrier formed by the polymers and results in transport of the coloured particles along/through the matrix thereby revealing the visual symbol.

Thus, it will be apparent to the skilled person, in view of the foregoing, that the embodiments of the invention described above in relation to a barrier formed by the cross-linked protease-sensitive polymers lend themselves to application in an *ex situ* lateral flow-based format. Thus, in a related aspect, the disclosure also provides:
A product for monitoring the condition of a wound comprising, consisting essentially of or consisting of:
a. a sample application zone to which wound fluid is added
b. a reaction zone downstream of the sample application zone comprising, consisting essentially of or consisting of cross-linked protease-sensitive polymers;
c. a zone comprising, consisting essentially of or consisting of coloured particles downstream of the reaction zone; and optionally
d. a viewing zone downstream of the sample application zone and reaction zone;
wherein the sample application zone transmits fluid towards the reaction zone and zone comprising, consisting essentially of or consisting of coloured particles (and viewing zone if present) and wherein the polymers in the reaction zone form a barrier that prevents wound fluid reaching the zone comprising, consisting essentially of or consisting of coloured particles (and the viewing zone if present). Cleavage of the polymers by protease activity present in the wound fluid, optionally above a threshold level, disrupts the barrier and results in carriage of the coloured particles with the wound fluid along/through the matrix thereby providing a visual indication of protease activity (which can be observed in the viewing zone if present). The embodiments described above in relation to a barrier formed by the cross-linked protease-sensitive polymers may apply to this lateral flow aspect *mutatis mutandis* and are not repeated here simply for conciseness.

According to all aspects and embodiments of the invention described herein, the (optionally cross-linked) protease-sensitive polymers comprise, consist essentially of or consist of cleavage sites for one or more proteases. Preferably, the protease-sensitive polymers comprise, consist essentially of or consist of cleavage sites for multiple proteases reflective of the proteases that are typically responsible for incorrect wound healing when present in excess (as discussed in greater detail herein). As a consequence, the (optionally cross-linked) protease-sensitive polymers may be intended to replicate the extracellular matrix environment of the wound, in particular the wound boundary. The reaction zone may thus replicate an extracellular matrix from a wound. Thus, the polymers may be considered in some embodiments as molecular "fibers", with the optional cross-links being formed between the fibers. The (optionally cross-linked) protease-sensitive polymers may form a hydrogel structure (e.g. a gelatin hydrogel). In particular embodiments, the polymers comprise, consist essentially of or consist of a protein/polypeptide. However, it will be apparent to the skilled person that synthetic polymers may also be suitable provided they comprise, consist essentially of or consist of one or more cleavage sites for at least one protease. In particular embodiments, the polymer may comprise, consist essentially of or consist of both naturally-occurring and non-naturally occurring monomer units. In specific embodiments, the polymer comprises, consists essentially of or consists of, optionally cross-linked, collagen. The collagen may form a collagen plaque. In preferred embodiments, the collagen is fully, substantially or partially denatured prior to use in the invention. Where this has occurred by partial hydrolysis of the collagen, it is termed "gelatin" as would be well-known to the person skilled in the art. Thus, in these preferred embodiments, the polymer comprises, consists essentially of or consists of, optionally cross-linked, gelatin. In other embodiments, the polymer comprises, consists essentially of or consists of, optionally cross-linked, elastin.

According to all aspects and embodiments of the invention described herein, the sensitivity to protease activity of the (optionally cross-linked) protease-sensitive polymers can be adjusted by multiple (combinable) means, directly and indirectly. Relevant factors include the length of time that the product will be placed in contact with wound fluid (i.e. governed by how long the reaction zone will be exposed to wound fluid). For instance, in some embodiments, the reaction zone comprising the (optionally cross-linked) protease-sensitive polymers further comprises one or more protease inhibitors. In specific embodiments, the one or more protease inhibitors may be (directly (e.g. covalently) or indirectly (e.g. by adsorption)) associated with the (optionally cross-linked) protease-sensitive polymers. The one or more protease inhibitor molecules present in the reaction zone inhibit specific proteases present in the wound fluid and therefore, in effect, reduce the levels of active protease present which is able to cleave the (optionally cross-linked) protease-sensitive polymers. For example, the one or more protease inhibitors may comprise, consist essentially of or consist of Ilomastat (GM6001, Galardin) which is a broad spectrum matrix metalloprotease (MMP) inhibitor for a range of MMPs, especially MMP-1 MMP-2, MMP-3, MMP-7, MMP-8, MMP-9, MMP-12, MMP-14 etc. and/or the elastase inhibitor Elastase Inhibitor-V from Calbiochem (2-(2-Bromophenyl)-5-chloro-3,1-benzoxazin-4-one), which is a benzoxazinone compound that acts as a potent inhibitor of human leukocyte elastase (IC₅₀ = 29.5 nM) by covalently modifying the active site serine via a Michael addition-elimination reaction. Another (indirect) means of tailoring the sensitivity to protease activity of the (optionally cross-linked) protease-sensitive polymers is to further include one or more sacrificial proteins/polypeptides in the reaction zone comprising the (optionally cross-linked) protease-sensitive polymers. The one or more sacrificial proteins/polypeptides may, for example, comprise, consist essentially of or consist of albumin and/or optimised (synthetic) protease-sensitive peptides. The one or more sacrificial proteins/polypeptides act as an alternate (competitive) substrate for proteases in the wound fluid thereby diverting protease activity away from the (optionally cross-linked) protease-sensitive polymers. For those embodiments in which the protease-sensitive polymers are cross-linked, a further direct means of tailoring the sensitivity of the polymers to protease activity is by increasing or decreasing the extent of cross-linking (as further described herein). Increased cross-linking will reduce protease sensitivity because the increased number of cross-links requires more extensive cleavage of the protease-sensitive polymers before the coloured particles are able to be transported along/through the matrix by the wound fluid. *Vice versa,* decreased cross-linking will increase protease sensitivity based on the same principle. Each of these means of manipulating the sensitivity to protease activity of the (optionally cross-linked) protease-sensitive polymers can be used alone or in combination, as appropriate (depending, for instance, on whether the protease-sensitive polymers are cross-linked or not).

According to all aspects and embodiments of the invention described herein, the cross-links formed between the polymer molecules may be direct or indirect. The inventors of the present invention have found that cross-linking of the polymer molecules advantageously stabilises the polymer molecules under aqueous conditions and prevents gradual dissolution of the polymers in the absence of (diagnostically relevant) protease activity, which is particularly pertinent in the context of products that are to be left in the wound for a significant period of time (e.g. under a wound dressing for more than a day). In addition, as described above, cross-linking enhances the resistance of the polymers to cleavage by protease activity unless and until protease activity is present in sufficient amounts to indicate a change in condition of the wound, which may be at or above a (pre-determined) threshold level of protease activity. Typically, the unmodified polymer molecules are first modified with one or more cross-linkable groups after which the modified polymer molecules are treated under suitable cross-linking conditions (as described elsewhere herein) to yield cross-linked polymers. Suitable cross-linking conditions depend on the cross-linkable group employed. For instance, suitable conditions may comprise, consist essentially of or consist of the use of ultraviolet (UV) radiation optionally in the presence of a suitable photoinitiator. The degree of modification of the unmodified polymer molecules with cross-linkable groups can be controlled by controlling the relative molar concentrations of polymer to cross- linkable groups and/or by suitably altering the reaction conditions for modification e.g. varying the pH and/or temperature of the reaction which may influence/alter the charge state of the monomer units from which the polymer is comprised, consists essentially of or consists of and/or conformation of the polymer. The degree of modification can be quantitated by suitable means known in the art such as by nuclear magnetic resonance spectroscopy. For those embodiments of the product suitable for use in *in situ* detection of the change in condition of a wound, maximum derivatisation of the polymer with the cross-linkable groups is preferred.

In preferred embodiments, the polymer molecules (typically gelatin, collagen and/or elastin) are modified with methacrylate, methacrylic anhydride or derivatives thereof after which the methacrylate, methacrylic anhydride or derivatives thereof are linked together under suitable conditions known to those skilled in the art. Thus, for these embodiments, the cross-links between polymers comprise, consist essentially of or consist of methacrylate or derivatives thereof. Cross-linking can be achieved under any suitable conditions which are known to those skilled in the art, for instance via exposure to UV radiation optionally in the presence of a suitable photoinitiator.

In other embodiments, the polymer molecules (typically gelatin, collagen and/or elastin) are modified with glutaraldehyde or derivatives thereof after/during which the glutaraldehyde or derivatives thereof are linked together under suitable conditions known to those skilled in the art. Thus, for these embodiments, the cross-links between polymers are derived from glutaraldehyde or derivatives thereof. Cross-linking can be achieved under any suitable conditions which are known to those skilled in the art.

As described herein, according to all aspects and embodiments of the invention, the polymers employed in the invention are protease-sensitive (i.e. they comprise, consist essentially of or consist of at least one protease cleavage site). In some embodiments, the polymers may be sensitive to one particular protease. In other more preferred embodiments, the polymers may be sensitive to multiple proteases (those reflective of *in vivo* wound healing processes). As the skilled person will appreciate, this will depend on the cleavage sites present in the polymer. Each cleavage site may be susceptible to cleavage by one specific protease or by multiple proteases. Alternatively, or in addition, the polymer may comprise, consist essentially of or consist of two or more different cleavage sites cleavable by different proteases. Generally, the cleavage sites comprise, consist essentially of or consist of a peptide sequence of two or more amino acid moieties which can be recognised by one or more specific proteases able to sever at least one peptide bond (between two amino acid moieties) in the cleavage site. Protease cleavage sites and reciprocal proteases are well-known in the art. The number of cleavage sites in the polymer should be such that, following cleavage of the polymers by protease activity present in the wound fluid, the polymers are sufficiently degraded to result in transport of the coloured particles along/through the matrix to provide a visual indication of protease activity in the wound fluid. The skilled person is well able to determine a sufficient number of cleavage sites using routine experimentation in view of this requirement. In specific embodiments, the cleavage sites are specifically recognised and cleavable by a serine protease, cysteine protease, aspartic protease, threonine protease and/or glutamic protease. In particular embodiments, the cleavage sites are specifically recognised and cleavable by one or more matrix metalloproteinases such as MMP2, MMP8 and/or MMP9. In preferred embodiments, the cleavage sites are specifically recognised and cleavable by collagenase, gelatinase and/or elastase enzymes (such as neutrophil elastase, more particularly human neutrophil elastase). In yet further embodiments, the cleavage sites are specifically recognised and cleavable by a cathepsin protease such as cathepsin G. In yet further embodiments, the cleavage sites are specifically recognised and cleavable by a papain-family enzyme, such as staphopain from *Staphylococcus aureus.* Advantageously, the protease-sensitive polymers can be cleaved (which may be referred to as "digested") by multiple proteases relevant to wound healing. The multiple proteases may be selected from, up to all of, the proteases listed above.

In certain embodiments, according to all aspects and other embodiments of the invention described herein, the protease-sensitive polymers are cleaved (to any significant degree) only if protease activity is present in the wound fluid at or above a pre-determined threshold level. In these embodiments, if protease activity is absent or present at levels below the threshold then, by virtue of a lack (to any significant degree) of cleavage of the polymers, a negligible amount or no coloured particles are transported along/through the matrix and, consequently, no visual indication of protease activity is provided. For instance, the threshold level of protease activity may be that which is expected in a healing wound. For example, some collagenase activity would be expected in a healing wound, but an excess of activity can indicate that the wound condition has deteriorated. In specific embodiments, the pre-determined threshold level may be in the range 0.0001-0.1 mg/mL. For instance, it may be 0.0001 mg/mL, 0.00015 mg/mL, 0.00031 mg/mL, 0.00062 mg/mL, 0.001 mg/mL, 0.00125 mg/mL, 0.0025 mg/mL, 0.005 mg/mL, 0.01 mg/mL, 0.0125 mg/mL, 0.025 mg/mL, 0.05 mg/mL or 0.1 mg/mL. The thresholds may apply to total protease activity detectable in the sample. In particular embodiments wherein the one or more proteases comprises, consists essentially of or consists of a collagenase/gelatinase, a matrix metalloproteinase such as MMP2, MMP8 and/or MMP9, neutrophil elastase (optionally human neutrophil elastase) and/or papain-family enzymes, such as staphopain from *Staphylococcus aureus,* the pre-determined threshold level may be 0.0001 mg/mL, 0.00015 mg/mL, 0.00031 mg/mL, 0.00062 mg/mL, 0.001 mg/mL, 0.00125 mg/mL, 0.0025 mg/mL, 0.005 mg/mL, 0.01 mg/mL, 0.0125 mg/mL, 0.025 mg/mL, 0.05 mg/mL or 0.1 mg/mL. In particular embodiments, the pre-determined threshold level of activity of a matrix metalloproteinase such as MMP2, MMP8 and/or MMP9 is in the range 0.0007-0.025 mg/mL. In particular embodiments, the pre-determined threshold level of activity of a matrix metalloproteinase such as MMP2, MMP8 and/or MMP9 is at or above 0.00076 mg/mL. In other embodiments, the pre-determined threshold level of activity of a matrix metalloproteinase such as MMP2, MMP8 and/or MMP9 is at or above 0.0228 mg/mL. The thresholds may apply to total matrix metalloproteinase activity detectable in the sample. In particular embodiments, the pre-determined threshold level of activity of a neutrophil elastase (optionally human neutrophil elastase) is in the range 0.0059-0.344 mg/mL. In specific embodiments, the pre-determined threshold level of activity of a neutrophil elastase (optionally human neutrophil elastase) is at or above 0.0995 mg/mL. The thresholds may apply to total neutrophil elastase activity detectable in the sample. In particular embodiments, the pre-determined threshold level of activity of a cathepsin protease such as cathepsin G is in the range 0.005-0.05 mg/mL. The thresholds may apply to cathepsin protease activity detectable in the sample.

Gelatin is a particularly suitable material for use in the reaction zones of the invention and provides an indicator of physiologically relevant protease activity in wound fluid. Thus, the disclosure also provides a product for monitoring the condition of a wound comprising, consisting essentially of or consisting of a matrix that absorbs wound fluid, the matrix comprising, consisting essentially of or consisting of:
a. cross-linked gelatin forming a reaction zone on/in the matrix; and
b. coloured (preferably polystyrene) microparticles;
wherein the cross-links comprise, consist essentially of or consist of: (i) methacrylate or derivatives thereof, or, (ii) glutaraldehyde or derivatives thereof; and wherein the arrangement of the polymers and coloured particles is such that cleavage of the polymers by gelatinase activity present in the wound fluid results in transport of the coloured particles along/through the matrix to provide a visual indication of gelatinase activity in the wound fluid. All features and embodiments described above, are relevant in relation to this product *mutatis mutandis* and are not repeated here simply for conciseness.

Similarly, the disclosure also provides a product for monitoring the condition of a wound comprising, consisting essentially of or consisting of:
a. a sample application zone to which wound fluid is added
b. a reaction zone downstream of the sample application zone comprising, consisting essentially of or consisting of cross-linked gelatin molecules wherein coloured (preferably polystyrene) microparticles are entrapped within the gelatin molecules; and optionally
c. a viewing zone downstream of the sample application zone and reaction zone;
wherein the cross-links comprise, consist essentially of or consist of: (i) methacrylate or derivatives thereof, or, (ii) glutaraldehyde or derivatives thereof; and
the sample application zone transmits fluid towards the reaction zone (and viewing zone if present) and wherein cleavage of the gelatin molecules by gelatinase activity present in the wound fluid, optionally above a threshold level, results in carriage of the coloured (polystyrene) microparticles with the wound fluid along/through the matrix thereby providing a visual indication of gelatinase activity (which can be observed in the viewing zone if present). All features and embodiments described above, are relevant in relation to this product *mutatis mutandis* and are not repeated here simply for conciseness.

Similarly, the disclosure also provides a product for monitoring the condition of a wound comprising, consisting essentially of or consisting of:
a. a sample application zone to which wound fluid is added
b. a reaction zone downstream of the sample application zone comprising, consisting essentially of or consisting of cross-linked gelatin molecules;
c. a zone comprising, consisting essentially of or consisting of coloured (preferably polystyrene) microparticles downstream of the reaction; and optionally
d. a viewing zone downstream of the sample application zone and reaction zone;
wherein the cross-links comprise, consist essentially of or consist of: (i) methacrylate or derivatives thereof, or, (ii) glutaraldehyde or derivatives thereof; and wherein the sample application zone transmits fluid towards the reaction zone and zone comprising, consisting essentially of or consisting of coloured (polystyrene) microparticles (and viewing zone if present) and wherein the gelatin molecules in the reaction zone form a barrier that prevents wound fluid reaching the zone comprising, consisting essentially of or consisting of coloured (polystyrene) microparticles (and the viewing zone if present). Cleavage of the gelatin molecules by gelatinase activity present in the wound fluid, optionally above a threshold level, disrupts the barrier and results in carriage of the coloured polystyrene microparticles with the wound fluid along/through the matrix thereby providing a visual indication of gelatinase activity (which can be observed in the viewing zone if present). All features and embodiments described above, are relevant in relation to this product *mutatis mutandis* and are not repeated here simply for conciseness.

All of the aforementioned products and embodiments may further comprise a housing to contain the matrix. Where a housing is provided it may comprise, consist essentially of or consist of one or more, optionally two, viewing windows that define one or more viewing zones on the matrix for viewing the coloured particles. These viewing windows may be positioned along the housing such that, prior to exposure to wound fluid and in the absence of protease activity, the coloured particles are visible in a first viewing window but are not visible in a second viewing window. For those embodiments comprising, consisting essentially of or consisting of one or more viewing zones, the one or more viewing windows may be aligned such that the viewing zones are visible through (and defined by) the viewing windows. Alternatively, the portions of the matrix that do not comprise, consist essentially of or consist of the reaction zone and/or viewing zone may be masked so that any coloured particles are not visible to the end user. This helps to simplify the signals to be interpreted.

More generally, in a related aspect, the disclosure also provides a test matrix comprising, consisting essentially of or consisting of collagen and/or gelatin polymers, the polymers comprising methacrylate cross-links or cross-links derived from glutaraldehyde. In preferred embodiments, the test matrix is suitable for measuring protease activity, preferably in wound fluid. The test matrix may comprise any one or more additional features as described herein in relation to the other aspects of the invention.

Similarly, the disclosure also provides methacrylate cross-linked gelatin polymers for use in measuring protease activity in wound fluid.

In a further aspect, the disclosure describes the use of cross-linked and protease-sensitive polymers as a substrate for measuring protease activity in wound fluid. In preferred embodiments, the polymers used comprise, consist essentially of or consist of collagen, gelatin and/or elastin polymers. Gelatin is most preferred. In further embodiments the cross-links comprise, consist essentially of or consist of methacrylate and/or are derived from glutaraldehyde.

In another aspect, the invention provides a method for monitoring the condition of a wound (of a subject) comprising:
a. applying a sample of wound fluid to a product as defined herein; and
b. detecting the visual indication of protease activity provided by the coloured particles.

In particular embodiments of the method, the coloured particles are measured in a region of the matrix to provide a quantitation of the protease activity in the wound fluid. In related embodiments, the region comprises, consists essentially of or consists of a viewing zone as defined elsewhere herein. In some embodiments, the product employed in the methods comprises, consists essentially of or consists of two viewing zones and loss of coloured particles in one zone is compared with gain in coloured particles in a second zone. For instance, prior to exposure to wound fluid and in the absence of protease activity, a first viewing zone may comprise, consist essentially of or consist of the coloured particles whilst the second viewing zone does not. Upon exposure to wound fluid, cleavage of the polymers by protease activity present in the wound fluid results in release of the coloured particles along/through the matrix. As a consequence, the coloured particles move from the first viewing zone along/through the matrix in the direction of flow of the wound fluid to the second viewing zone. In preferred embodiments, the change in colour in each viewing zone is visible to the naked eye. Alternatively, or in addition to, observance via the naked eye, the change in colour can be detected and/or quantified using a spectrophotometer or other such equipment suitable for this purpose, which are well-known to the skilled person.

In some embodiments, the method further comprises, consists essentially of or consists of applying additional liquid to the product to assist with fluid flow of the wound fluid. This may be termed a "chase fluid". The additional liquid may comprise, consist essentially of or consist of a buffer. The buffer may be matched to the properties of the wound fluid and/or other materials employed in the method. For example, a "chase fluid" may be employed in order to carry the wound fluid through the pores of a test strip. Use of additional liquid can improve transmission of released coloured particles along/through the matrix. The additional liquid may comprise, consist essentially of or consist of a surfactant to prevent unwanted adhesion of the coloured particles to the matrix.

In another aspect of the invention, the methods described herein are repeated at intervals in order to facilitate longitudinal monitoring of the condition of the wound by repeated sampling and analysis of the wound fluid. Said intervals may be every ½, 1, 2, 3, 4, 5 or 6 days, weekly or monthly or a combination thereof. The aggregation of data pertaining to the condition of the wound over time better enables the clinician to understand the progress of the condition of the wound and/or efficacy of treatment(s). For instance, longitudinal monitoring of the wound fluid as described may indicate to the clinician, in a more rapid and/or quantitative fashion than current procedures, that the condition of the wound is deteriorating over time and thus the present treatment is ineffective. Consequently, the clinician can more rapidly select alternative treatments in order to promote healing of the wound. Alternatively, the data may indicate to the clinician that further tests of the wound fluid and/or wound environment are needed.

In a further embodiment, the absence of excess protease activity indicates that any existing treatment of the wound should be continued (i.e. should not be altered).

Also described herein is a process of making a matrix for measuring protease activity in wound fluid, comprising, consisting essentially of or consisting of:
a. Applying a solution containing methacrylate-modified polymers to a matrix capable of absorbing wound fluid; and
b. Irradiating the polymers with UV light thereby cross-linking the polymers.

The method thus produces a matrix comprising, consisting essentially of or consisting of a reaction zone. The reaction zone typically covers a discrete portion of a visible surface of the matrix and thus does not saturate the matrix.

Methacrylate-modified polymers can be prepared using any suitable technique/protocol known in the art. For instance, where the polymer comprises, consists essentially of or consists of amine groups, such as lysine ε-amine groups in a protein/polypeptide, the polymer can be reacted with methacrylic anhydride which may be in (high) excess. According to the method of Be Hoon Lee et al. (RSC Adv., 2015, 5, 106094-106097), this reaction can be performed at approximately 50°C for approximately 3 hours in carbonate buffer to maintain the pH at approximately pH 7.0-9.0, adjusted with methacrylic anhydride and sodium hydroxide as necessary. This method allows for efficient modification of lysine ε-amine groups in a protein/polypeptide whilst limiting unwanted side-reactions. The methacrylate-modified polymers produced can be purified using suitable methods known in the art such as tangential flow filtration or dialysis.

In particular examples, the solution of step a) also contains coloured particles and step b) results in entrapment of the coloured particles within the cross-linked polymers.

In further examples, the solution of step a) further comprises a suitable photo initiator (to begin/catalyse the cross-linking reaction upon exposure to UV irradiation in step (b)). In particular examples, the final concentrations of photo initiator, methacrylate-modified polymers and coloured particles in the solution of step (a) are in the range: 0.01-10% (w/v) photo initiator, 0.01-50% (w/v) methacrylate-modified polymers and 0.01-40% (w/v) coloured particles. In some examples, the final concentrations of photo initiator, methacrylate-modified polymers and coloured particles in the solution of step (a) are in the range: 0.1-1% (w/v) photo initiator, 1-10% (w/v) methacrylate-modified polymers and 0.05-5% (w/v) coloured particles. In preferred examples, the final concentrations of photo initiator, methacrylate-modified polymers and coloured particles in the solution of step (a) are: 0.45% photo initiator, 8.1% methacrylate-modified polymers and 0.25% coloured particles.

In further examples, the solution is applied at a temperature of at least 30°C. This helps to prevent solidification of the methacrylate-modified polymers until desired. In some embodiments, a thickening agent (such as carboxy methyl cellulose or gelatin) may be added to the solution to increase its viscosity so as to ease manipulation of the solution onto the matrix prior to UV irradiation.

In particular examples, the volume of solution containing methacrylate-modified polymers which is applied to the matrix is in the range of 1-10 µl, 1-20 µl, 1-50 µl, 1-100 µl or 1-1000 µl. In particular embodiments, this volume is 5 µl. In other examples, this volume is 1 µl.

In particular examples, the polymers are irradiated with UV light for approximately 15-30 seconds, optionally in the presence of a photoinitiator. In some embodiments, the cross-linked polymers are then dried on/in the matrix. This may be, for instance, by air-drying, optionally for 1-5 hours (preferably 3 hours).

In preferred embodiments, the methacrylate-modified polymers comprise, consist essentially of or consist of gelatin, collagen and/or elastin polymers.

In further embodiments, the matrix comprises a visual symbol and in step a) the solution is applied so as to cover the visual symbol. The visual symbol may be printed on the matrix using any suitable means (e.g. indelible ink).

As the skilled person will appreciate, the aforementioned methods can be used to make a matrix for use in a product as defined herein.

Also disclosed is a kit for making a product as defined herein comprising, consisting essentially of or consisting of:
a. cross-linked and protease-sensitive polymers (preferably gelatin, collagen and/or elastin polymers); and
b. coloured particles.

In particular examples of the disclosure, the kit further comprises a matrix as described herein. The kit may also contain a housing to contain the matrix as described herein. Where a housing is provided it may comprise, consist essentially of or consist of one or more, optionally two, viewing windows for viewing the coloured particles. These viewing windows may be positioned along the housing such that, prior to exposure to wound fluid and in the absence of protease activity, the coloured particles are visible in a first viewing window but are not visible in a second viewing window.

While it is envisaged that the most advantageous *in situ* application of the products of the invention is as a discrete product packaged entirely separately from a wound dressing, it is also possible to integrate the products of the invention into a wound dressing. Thus, the disclosure also provides a wound dressing incorporating a product of the invention as defined herein. The disclosure includes that a wound dressing and product may be provided in a kit of parts. Thus, the wound dressing incorporates the product of the invention when placing the wound dressing on the wound, as described in further detail herein.

It will be apparent to the skilled person that the products described herein can be designed or employed so as to absorb enough wound fluid to be able to provide a visual indication protease activity in the wound fluid without necessarily absorbing, or being able to absorb, sufficient wound fluid for further downstream processing as described herein. Thus, in certain embodiments, the product functions solely as an in-wound protease activity detector. These embodiments are advantageous as they are extremely simple to operate and interpret.

However, it will also be apparent to the skilled person from the present disclosure that a key aspect of many embodiments of the product of the invention for *in situ* application is the ability to absorb sufficient wound fluid to enable further laboratory based testing of the fluid, in a remote setting from the subject. Once removed from the wound, the product then needs to be safely delivered to the laboratory in a manner such that the fluid remains diagnostically useful. Thus, described herein is a kit comprising, consisting essentially of or consisting of a product as described herein and a vessel (suitable) for safe containment and shipping of the product. Following contact of the product with the wound and absorbance of wound fluid, the product can be removed from the wound and placed in the vessel to allow safe transportation to a laboratory for further analysis of the wound fluid, as further described herein.

In embodiments of the product wherein the matrix comprises, consists essentially of or consists of a first and second portion as described herein, the first and second portion may be provided as two separate components in the kits described herein. In particular embodiments, these two components may be connectable to each other such that the user at the point of care can assemble the two components into a single unit for placing into contact with the wound (fluid). The wound dressing may retain the single unit in place. In certain embodiments, where the second portion is able to and has absorbed wound fluid in an amount sufficient for downstream analysis of the wound fluid as described further herein, only this second portion need be safely delivered to the laboratory as described herein. Thus, the second portion may be detachable from the first. Alternatively, the whole unit may be sent for further testing. The test result in the first portion provides useful diagnostic information so it may be advantageous to also transmit this.

In certain embodiments, at least the internal surface(s), and generally all surfaces to include external surfaces, of the containment vessel are biologically inert.

In further embodiments, contact of the matrix with the internal surface or surfaces of the containment vessel does not measurably alter the condition of the fluid or its components (markers).

The vessel should be sealable to enable safe transport without risk of the fluid leaking. The seal may be reversible or may need to be broken at the laboratory in order to gain access to the fluid. The vessel is typically a consumable single use item. It may be made of plastic in some embodiments. It may, however, be reusable following suitable sterilisation e.g. by autoclaving in some embodiments.

Described herein by way of example only is a method for monitoring the condition of a wound on a subject comprising, consisting essentially of or consisting of:
(a) placing a product of the invention as described herein in contact with the wound under a wound dressing;
(b) leaving the product in contact with the wound for a pre-determined amount of time;
(c) determining the presence or absence of a visual indication of protease activity in the wound by the product;
wherein the presence of the visual indication signals the need for further analysis of the wound fluid.

In embodiments of the product wherein the matrix comprises, consists essentially of or consists of a first and second portion as described herein, the first and second portions may be separate components, not connected to each other and independently placed in contact with the wound for a pre-determined amount of time. In other embodiments, the first and second portions may be provided as separate components which are connectable with one another and assembled by the user at the point of care into a single unit. This single unit is then placed in contact with the wound for a pre-determined amount of time. Thus, the second portion may be detachable from the first. Alternatively, the whole unit may be sent for further testing. The test result in the first portion provides useful diagnostic information so it may be advantageous to also transmit this. In particular embodiments, the first portion comprises, consists essentially of or consists of the cross-linked and protease-sensitive polymers described herein on or in that portion of the matrix. The first portion is able to absorb sufficient wound fluid to expose the cross-linked and protease-sensitive polymers comprised on or in the first portion to the wound fluid and, therefore, the protease activity that may be contained therein. Thus, this first portion provides the visual indication of protease activity in the wound by the product as described herein. The second portion is able to absorb wound fluid in an amount sufficient for downstream analysis of the wound fluid as described further herein.

However, the visual indication may be combined with one or more indications selected from:
(i) the smell of the wound
(ii) the total volume of fluid
(iii) the appearance of the wound
(iv) the systemic condition of the subject
in order to determine the need for further analysis of the wound fluid.

The absence of a visual indication by the product may be offset by the presence of one or more of the other indications listed above which, when assessed collectively, determines that further analysis of the wound fluid is needed. Such assessment may be made by a caregiver, such as a district nurse, at the point of care or a clinician.

By way of example only, the method may further comprise, consists essentially of or consists of:
(d) removal of the product from contact with the wound;
(e) retrieving the fluid absorbed by the product;
(f) analysing the retrieved fluid in order to determine the condition of the wound.

Removal of the product may utilise forceps or another instrument to prevent direct human contact with the matrix.

Where the product comprises, consists essentially of or consists of a matrix comprising, consisting essentially of or consisting of a first and second portion as described herein, the wound fluid may be retrieved from the second matrix portion which has absorbed an amount of wound fluid sufficient for downstream analysis of the wound fluid as described further herein.

By way of example only, step (d) may further comprise, consists essentially of or consists of storage and shipping of the product to a laboratory before step (e) is performed. For embodiments of the product wherein the matrix comprises, consists essentially of or consists of a first and second portion as described herein, in certain examples only the second matrix portion is stored and shipped to the laboratory as it is this portion which has absorbed an amount of wound fluid sufficient for downstream analysis of the wound fluid as described further herein. In other examples, both the first and second matrix portion are stored and shipped to the laboratory so that, for instance, the extent of degradation of the one or more markers present in the first matrix portion can be evaluated in the laboratory. Thus, the second portion may be detachable from the first. Alternatively, the whole unit may be sent for further testing. The test result in the first portion provides useful diagnostic information so it may be advantageous to also transmit this. Storage of the product may be in a vessel as described herein suitable for safe containment and shipping prior to steps (e) and (f). The internal and external surfaces of the containment vessel may be biologically inert. In further examples, contact of the matrix with the internal surface of the containment vessel does not measurably alter the condition of the fluid or its components.

In a further example, the vessel containing the product removed from the wound is transported to a laboratory for further analysis of the absorbed wound fluid.

Retrieval of the fluid may be by any suitable means. The matrix may be squeezed to release the fluid or may be centrifuged for example.

Analysis of the retrieved fluid comprises, consists essentially of or consists of one or more tests to characterise the condition of the wound. Such tests may facilitate treatment and selection of therapeutic and other clinical interventions. Any suitable test may be employed as would be readily understood by one skilled in the art. In certain embodiments, analysis of the retrieved fluid comprises, consists essentially of or consists of measuring the levels and/or activities of one or more of:
(i) *N*-terminal serum type 1 procollagen (P1NP)
(ii) *N*-acetyl-Proline-Glycine-Proline (acPGP)
(iii) a biomarker of neutrophil infiltration

N-terminal serum type 1 procollagen (P1NP) is an indicator molecule of collagen synthesis. *N*-acetyl-Proline-Glycine-Proline (acPGP) is a degradation product of collagen produced as a consequence of proteolytic cleavage of collagen by matrix metalloproteinases, in particular collagenase. Consequently, in a further embodiment, the levels of P1NP and acPGP are used to determine a Healing Index Ratio wherein:
(i) equal levels of P1NP and acPGP indicate healing and/or successful treatment of the wound because the rates of collagen synthesis and degradation are approximately in balance;
(ii) higher levels of acPGP relative to P1NP indicates inflammation and/or increased risk of infection within the wound because more collagen is being degraded than is being synthesised;
(iii) moderately higher levels of P1NP relative to acPGP indicates a strong healing trajectory because more collagen is being synthesised than is being degraded;
(iv) significantly higher levels of P1NP relative to acPGP indicates an increased risk of hypergranulation in the wound because excessive amounts of collagen are being synthesised relative to the rates of collagen degradation.

"Hypergranulation" is to be understood as the excessive deposition of granulation tissue that may extend above the wound margin and comprises, consists essentially of or consists of newly formed collagen, elastin and capillary networks.

During wound healing, infiltrating neutrophils are recruited to the wound site and are involved in tissue degradation and tissue formation. As such, an excessive or reduced influx or activation of infiltrating neutrophils into the damaged tissue may have profound effects on downstream cell migration, proliferation, differentiation, and ultimately the quality of the healing response. In particular, calprotectin is a protein produced by neutrophils known to be present in plasma and markedly elevated in inflammatory conditions. Thus, in certain embodiments, the biomarker of neutrophil infiltration is calprotectin.

In further embodiments, analysis of the retrieved fluid further or alternatively comprises, consists essentially of or consists of measuring the levels of one or more of:
(i) Hydroxylation of lysine and proline residues free in the wound fluid;
(ii) angiogenesis biomarkers; and/or
(iii) blood vessel differentiation biomarkers

In certain embodiments, low levels of hydroxylation of lysine and proline residues free in the wound fluid indicate the need for treatment to promote increased blood flow and access of oxygen to the wound.

In certain embodiments, the angiogenesis biomarkers comprise, consist essentially of or consist of, consist essentially of or consist of vascular endothelial growth factor.

In certain embodiments, the blood vessel differentiation biomarkers comprise, consist essentially of or consist of, consist essentially of or consist of intercellular adhesion molecule.

In certain embodiments, low levels of vascular endothelial growth factor and/or intercellular adhesion molecule indicate to treat the wound with externally applied vascular endothelial growth factor supplements.

In further embodiments, analysis of the retrieved fluid further or alternatively comprises, consists essentially of or consists of determining the nitric oxide (NO) status of the wound by measuring the levels and/or activities of one or more of:
(i) inducible nitric oxide synthase
(ii) carboxymethyllysine
(iii) arginase

As understood in the art, a lack of inducible nitric oxide synthase would disable NO responses normally needed in physiological homeostasis. Similarly, accumulation of carboxymethyllysine indicates that the wound site is in a NO deficient state. It will also be appreciated that arginase consumes arginine in a metabolic pathway which does not produce NO and is a competitor to NO synthase.

A low nitric oxide status indicates to treat the subject suffering from the wound with nitric oxide therapy or an arginine dietary supplement.

In further embodiments, analysis of the retrieved fluid further comprises, consists essentially of or consists of measuring the levels of one or more of the following markers:
(i) desmosine
(ii) matrix metalloproteinase (MMP8)
(iii) calprotectin
(iv) TIMP1
(v) TIMP2
(vi) A1AT
(vii) interleukin 6

By marker levels is meant the level of expression and/or activity and/or amount and/or concentration of the marker in the wound fluid. Expression levels may correlate with activity and can thus be used as a surrogate of activity and vice versa.

The person skilled in the art will be familiar with techniques and methods to determine the level of P1NP, acPGP, hydroxylation of lysine and proline residues, angiogenesis, vascular endothelial growth factor, blood vessel differentiation, intercellular adhesion molecule, inducible nitric oxide synthase, carboxymethyllysine, arginase, desmosine, MMP8, calprotectin, TIMP1, TIMP2, A1AT or interleukin 6.

For instance, expression levels may be measured at the level of protein or mRNA according to any suitable method. Protein modifications, such as glycosylation may also be relevant and can be measured by any suitable method. Many such methods are well known in the art and include use of mass spectrometry (e.g. MALDI-TOF mass spectrometry).

The expression level and/or amount and/or concentration of a marker (e.g. a protein) may rely upon a binding reagent such as an antibody or aptamer that binds specifically to the marker of interest (e.g. protein). The antibody may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Such antibodies are useful in the methods of the invention. They may be used to measure the level of a particular marker (e.g. protein, or in some instances one or more specific isoforms of a protein. The skilled person is well able to identify epitopes that permit specific isoforms to be discriminated from one another).

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain embodiments the expression level and/or amount and/or concentration of a marker is determined using an antibody or aptamer conjugated to a label. By label is meant a component that permits detection, directly or indirectly. For example, the label may be an enzyme, optionally a peroxidase, or a fluorophore.

A label is an example of a detection agent. By detection agent is meant an agent that may be used to assist in the detection of the antibody-marker (e.g. protein) complex. Where the antibody is conjugated to an enzyme the detection agent may comprise, consist essentially of or consist of a chemical composition such that the enzyme catalyses a chemical reaction to produce a detectable product. The products of reactions catalysed by appropriate enzymes can be, without limitation, fluorescent, luminescent, or radioactive or they may absorb or reflect visible or ultraviolet light. Examples of detectors suitable for detecting such detectable labels include, without limitation, x-ray film, radioactivity counters, scintillation counters, spectrophotometers, colorimeters, fluorometers, luminometers, photodetectors and densitometers. In certain embodiments the detection agent may comprise, consist essentially of or consist of a secondary antibody. The expression level is then determined using an unlabelled primary antibody that binds to the target protein and a secondary antibody conjugated to a label, wherein the secondary antibody binds to the primary antibody.

Additional techniques for determining expression level at the level of protein and/or the amount and/or concentration of a marker include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies. Levels of protein may be detected using a lateral flow assay in some embodiments.

Measuring mRNA in a biological sample may be used as a surrogate for detection of the level of the corresponding protein in the wound fluid. Thus, the expression level of any of the relevant markers described herein can also be detected by detecting the appropriate RNA.

Accordingly, the expression level may be determined by microarray, northern blotting, sequencing (including next generation sequencing, such as RNAseq) or nucleic acid amplification. Nucleic acid amplification includes PCR and all variants thereof such as real-time and end point methods and qPCR. Other nucleic acid amplification techniques are well known in the art, and include methods such as NASBA, 3SR and Transcription Mediated Amplification (TMA). Other suitable amplification methods include the ligase chain reaction (LCR), selective amplification of target polynucleotide sequences (US Patent No. 6,410,276), consensus sequence primed polymerase chain reaction (US Patent No 4,437,975), arbitrarily primed polymerase chain reaction (WO 90/06995), invader technology, strand displacement technology, recombinase polymerase amplification (RPA), nicking enzyme amplification reaction (NEAR) and nick displacement amplification (WO 2004/067726). This list is not intended to be exhaustive; any nucleic acid amplification technique may be used provided the appropriate nucleic acid product is specifically amplified. Design of suitable primers and/or probes is within the capability of one skilled in the art. Various primer design tools are freely available to assist in this process such as the NCBI Primer-BLAST tool. Primers and/or probes may be at least 15, 16, 17, 18, 19, 20, 21, 22, 23, 24 or 25 (or more) nucleotides in length. mRNA expression levels may be measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

RNA expression may be determined by hybridization of RNA to a set of probes. The probes may be arranged in an array. Microarray platforms include those manufactured by companies such as Affymetrix, Illumina and Agilent. RNA expression may also be monitored using next generation sequencing techniques, such as RNA-seq.

Similarly, activity of the one or more markers (e.g. enzymatic activity) may be measured in the wound fluid. Enzymatic activity may be measured for example by detecting processing of a substrate, which may be labelled. For example, the assay may be a fluorogenic substrate assay. Enzyme activity may be detected using a suitable lateral flow assay. Examples of suitable assay formats include the assays set forth in International Patent Applications WO2009/024805, WO2009/063208, WO2007/128980, WO2007/096642, WO2007/096637, WO2013/156794 and WO2013/156795.

The methods described herein may be repeated at intervals in order to facilitate longitudinal monitoring of the condition of the wound by repeated sampling and analysis of the wound fluid. Thus, for instance, following removal of a product as described herein which has been in contact with the wound for a pre-determined period of time and which has absorbed wound fluid and prior to re-dressing the wound, a new, sterile product as described herein is placed in contact with the wound underneath the new wound dressing and the method repeated in respect of the new product now in contact with the wound. Said intervals may be every ½, 1, 2, 3, 4, 5 or 6 days, weekly or monthly or a combination thereof. Said intervals may also be 24hr, 48hr, 120hr or 144 hr or any combination thereof. As discussed herein, the product may only be sent to the laboratory for further testing if the visual indication of protease activity in the wound fluid (i.e. an alteration in the condition of the wound) is provided. Nonetheless, by using a new product each time the wound is redressed, the initial marker testing is performed on a regularly repeated basis with the consequential benefit that the sample for downstream testing is being obtained on each occasion.

Longitudinal monitoring of the wound fluid in this manner may also be performed even in the absence of a visual indication by the product following contact with the wound for a pre-determined period of time ("pre-determined contact time"). For instance, the product may still be removed from contact with the wound (and replaced with a new, sterile product) and the absorbed wound fluid analysed as described herein if a pre-determined period of time has passed since fluid from the wound has been sampled ("the pre-determined sampling time"). The pre-determined sampling time may be every 1, 2, 3, 4, 5 or 6 days, weekly or monthly or a combination thereof. The pre-determined sampling time may also be every 24hr, 48hr, 120hr or 144 hr or any combination thereof. In a preferred embodiment, the pre-determined sampling time is 4 weeks after the previous sample was taken (in the absence of a visual indication from the product in the intervening period). Thus, for example, the product may be replaced each time the wound is dressed at or around 3 to 4 day intervals. The product may be sent to the laboratory as a matter of routine once a month even if none of the products have shown the visual indication in the intervening period.

The aggregation of data pertaining to the condition of the wound over time via sampling and analysing the wound fluid over time better enables the clinician to understand the progress of the condition of the wound and/or efficacy of treatment(s). For instance, longitudinal monitoring of the wound fluid as described may indicate to the clinician, in a more rapid and/or quantitative fashion than current procedures, that the condition of the wound is deteriorating over time and thus the present treatment is ineffective. Consequently, the clinician can more rapidly select alternative treatments in order to promote healing of the wound. Alternatively, the data may indicate to the clinician that further tests of the wound fluid and/or wound environment are needed. Furthermore, the aggregated data allows the clinician to develop a visiting schedule in relation to further sampling of the wound fluid and re-dressing of the wound by a caregiver, such as a district nurse or family member, depending on the degree and direction of change in the condition of the wound over time.

In a further embodiment, the absence of excess protease activity in the wound fluid indicates that existing treatment of the wound should be continued (i.e. should not be altered).

In another related aspect of the invention, a product is provided comprising (optionally cross-linked) protease-sensitive polymers for *ex situ* monitoring of the condition of the wound. The product allows lateral flow-based detection of protease activity, optionally at or above a (pre-determined) threshold level, in wound fluid. The product allows rapid, sensitive, *ex situ* point-of-care detection. As a consequence, exposure of the protease-sensitive polymers to wound fluid is typically for a lesser amount of time.

Thus, according to this aspect of the invention and as defined in the claims, a product for monitoring the condition of a wound is provided comprising:
a. a sample application zone to which wound fluid is added;
b. a reaction zone downstream of the sample application zone comprising (optionally cross-linked) protease-sensitive polymers;
c. a zone comprising coloured particles downstream of the sample application zone; and
d. a viewing zone downstream of the sample application zone, reaction zone and zone comprising coloured particles;
wherein the sample application zone transmits fluid towards the viewing zone and wherein cleavage of the polymers by protease activity present in the wound fluid results in carriage of the coloured particles with the wound fluid to the viewing zone thereby providing a visual indication of protease activity in the viewing zone,
wherein:
(i) the coloured particles are entrapped within the polymers and cleavage of the polymers by protease activity present in the wound fluid results in release of the coloured particles to the viewing zone thereby providing a visual indication of protease activity in the wound fluid via the viewing zone; or
(ii) the reaction zone forms a barrier that prevents wound fluid reaching the viewing zone and wherein cleavage of the polymers by protease activity present in the wound fluid, optionally above a threshold level, disrupts the barrier and results in carriage of the coloured particles to the viewing zone thereby providing a visual indication of protease activity in the wound fluid via the viewing zone.

Prior to exposure to wound fluid and in the absence of protease activity the viewing zone does not contain coloured particles. Advantageously, therefore, the transmission of coloured particles into the viewing zone in the presence of protease activity in the wound fluid provides a positive indication to the user of protease activity in the wound fluid. In preferred embodiments, the change and/or intensification in colour in the viewing zone is visible to the naked eye. Alternatively, or in addition to, observance via the naked eye, the change and/or intensification in colour can be detected and/or quantified using a spectrophotometer or other such equipment suitable for this purpose, which are well-known to the skilled person.

Transmission of wound fluid between zones may be by any suitable means, for instance via capillary action. Note, throughout the disclosure, the zones are defined with the product in a state before exposure to wound fluid. The products thus rely upon directional flow of wound fluid from the sample application zone, via the reaction zone and zone comprising, consisting essentially of or consisting of coloured particles, to the viewing zone. The product thus provides a solid support on which the sample application zone, reaction zone, zone comprising, consisting essentially of or consisting of coloured particles and viewing zone are arranged. Any suitable solid support may be employed. For example, the product may comprise, consist essentially of or consist of a test strip or capillary flow device as the solid support. The solid support may comprise, consist essentially of or consist of a chromatographic medium. The solid support may be made from any material through which a fluid is capable of passing, such as a fluidic channel or porous membrane. In certain embodiments of the invention, the chromatographic medium comprises, consists essentially of or consists of a strip or membrane, for example a nitrocellulose strip or membrane. In the case of a capillary flow device, one or more of the sample application zone, reaction zone, coloured particles zone and viewing zone are located in discrete chambers connected by capillary channels. Products of the invention may rely on combinations of test strip and capillary flow channels in some embodiments. For example, the sample application zone may be comprised, consists essentially of or consists of an absorbent material and wound fluid is then passed along to the reaction zone. Downstream of the reaction zone the solid support may define a narrow (compared to the sample application zone and/or reaction zone) capillary channel to assist with concentration of the coloured particles in the viewing zone. In some embodiments, the product comprises, consists essentially of or consists of a matrix, as described herein, on which the sample application zone, reaction zone, zone comprising, consisting essentially of or consisting of coloured particles and viewing zone are arranged.

In particular embodiments of the invention, the test strip or capillary flow device may comprise one or more debossed capillary flow channels. For instance, the test strip or capillary flow device comprising one or more debossed capillary flow channels may be formed of two (optionally plastic) pieces, each piece comprising a face with one or more debossed regions/cavities, the two pieces being joined together (e.g. by ultrasonic welding or adhesive) and arranged such that the debossed regions/cavities on each face form the one or more capillary flow channels. Alternatively, the test strip or capillary flow device may comprise a single (optionally plastic) piece comprising a face with one or more debossed regions/cavities. Over said one or more debossed regions/cavities is laid an adherent layer which is joined to said face comprising the one or more debossed regions/cavities, thus forming the one or more capillary flow channels. The layer may be a flat foil or film layer for example. It may be joined to the face comprising the one or more debossed regions/cavities by any appropriate means, for example, by adhesive or by ultrasonic welding.

In particular embodiments, the coloured particles are comprised within the reaction zone. Thus, the zone comprising, consisting essentially of or consisting of coloured particles is subsumed within the reaction zone (i.e. they are one and the same overall "zone").

In further embodiments, the coloured particles are entrapped within the polymers. This may be achieved by drying the polymers with the coloured particles. As a consequence, the polymers adopt the colouration of the entrapped particles. The coloured particles remain entrapped within the polymers until and unless the polymers are cleaved by protease activity present in wound fluid. After the addition of wound fluid to the sample application zone, the wound fluid is transmitted to the reaction zone. Cleavage of the polymers by protease activity present in the wound fluid results in release of the coloured particles to the viewing zone thereby providing a visual indication of protease activity in the wound fluid via the viewing zone.

For those embodiments in which the coloured particles are entrapped within the cross-linked, protease-sensitive polymers, this may be achieved by drying the polymers with the coloured particles. As a consequence, the polymers adopt the colouration of the entrapped particles. The coloured particles remain entrapped within the polymers unless and until the polymers are cleaved by protease activity present in the wound fluid. Following cleavage of the polymers by protease activity present in the wound fluid, the coloured particles are transported along/through the matrix to provide a visual indication of protease activity in the wound fluid. As described elsewhere herein, in some preferred embodiments, the cross-linking agent is methacrylate or derivatives thereof. The polymer molecules (typically gelatin, collagen and/or elastin) are modified with methacrylate, methacrylic anhydride or derivatives thereof after which the methacrylate, methacrylic anhydride or derivatives thereof are linked together under suitable conditions known to those skilled in the art. Thus, for these embodiments, the cross-links between polymers comprise, consist essentially of or consist of methacrylate or derivatives thereof. In other embodiments, glutaraldehyde or derivatives thereof may be used as the cross-linking agent. Cross-linking can be achieved under any suitable conditions which are known to those skilled in the art. Polymer molecules comprising, consisting essentially of or consisting of gelatin are most preferred.

Thus, the disclosure also provides a product for monitoring the condition of a wound comprising, consisting essentially of or consisting of:
a. a sample application zone to which wound fluid is added;
b. a reaction zone downstream of the sample application zone comprising, consisting essentially of or consisting of coloured particles entrapped within (optionally cross-linked) protease-sensitive polymers; and
c. a viewing zone downstream of the sample application zone and reaction zone;
wherein the sample application zone transmits fluid towards the viewing zone and wherein cleavage of the polymers by protease activity present in the wound fluid results in carriage of the coloured particles with the wound fluid to the viewing zone thereby providing a visual indication of protease activity in the first viewing zone.

In alternative embodiments, the zone comprising, consisting essentially of or consisting of coloured particles is downstream of the reaction zone i.e. it is distinct from the reaction zone and is positioned between the reaction zone and viewing zone.

Thus, the disclosure also provides a product for monitoring the condition of a wound comprising, consisting essentially of or consisting of:
a. a sample application zone to which wound fluid is added;
b. a reaction zone downstream of the sample application zone comprising, consisting essentially of or consisting of (optionally cross-linked) protease-sensitive polymers;
c. a zone comprising, consisting essentially of or consisting of coloured particles downstream of the reaction zone;
d. a viewing zone downstream of the sample application zone, reaction zone and zone comprising, consisting essentially of or consisting of coloured particles;
wherein the sample application zone transmits fluid towards the viewing zone and wherein cleavage of the polymers by protease activity present in the wound fluid results in carriage of the coloured particles with the wound fluid to the viewing zone thereby providing a visual indication of protease activity in the first viewing zone
In particular embodiments, the reaction zone and/or (optionally cross-linked) protease-sensitive polymers comprised, consisting essentially or consisting therein forms a barrier that prevents (in the absence of sufficient protease activity contained therein) wound fluid passing, more specifically prevents wound fluid reaching zones downstream of the reaction zone (i.e. the zone comprising, consisting essentially of or consisting of coloured particles and the viewing zone). Upon exposure to wound fluid, cleavage of the polymers by (sufficient) protease activity present in the wound fluid, optionally above a threshold level, disrupts the barrier and results in carriage of the coloured particles to the viewing zone thereby providing a visual indication of protease activity in the wound fluid via the viewing zone. Thus, for those embodiments in which the zone comprising, consisting essentially of or consisting of coloured particles is downstream of the reaction zone, the coloured particles are prevented from coming into contact with wound fluid unless and until the polymers are cleaved by protease activity present in the wound fluid, optionally above a threshold level, and the barrier is disrupted.

In some embodiments, the product comprises a visual symbol that, prior to exposure to wound fluid and in the absence of protease activity, is masked by the coloured particles. For instance, the visual symbol may be printed on the matrix using any suitable means (e.g. indelible ink) over which the coloured particles are initially deposited. After exposure to wound fluid, cleavage of the polymers by protease activity present in the wound fluid results in transport of the coloured particles towards the viewing zone thereby revealing the visual symbol.

In further embodiments, the product further comprises a second viewing zone aligned with (e.g. above and/or defined by) the zone comprising, consisting essentially of or consisting of coloured particles such that the coloured particles are visible in the second viewing zone prior to exposure to wound fluid and in the absence of protease activity. This additional viewing zone is referred to herein as "the second viewing zone". For such embodiments, the remainder of the product may be masked such that only the viewing zones are able to afford a clear visual indication to the user.

In further embodiments the viewing zone downstream of the sample application zone, reaction zone and zone comprising, consisting essentially of or consisting of coloured particles comprises, consists essentially of or consists of capture molecules to capture coloured particles in the viewing zone. This may assist with generation of an easily interpreted signal. The capture molecules may be arranged to produce a visible line or other symbol that may permit some level of quantitation of the signal (and thus of protease activity). The capture molecules are any molecules capable of binding to the coloured particles. These may comprise, consist essentially of or consist of, for instance, an antibody or aptamer that binds specifically to the coloured particles. In specific embodiments, the capture molecules are antibodies that bind specifically to the coloured particles. The antibodies may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Methods of generating specific antibodies and aptamers are well-known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In further embodiments, the product comprises a barrier at the downstream end of the viewing zone which is downstream of the sample application zone, reaction zone and zone comprising, consisting essentially of or consisting of coloured particles. That is to say that the viewing zone is positioned between the zone comprising, consisting essentially of or consisting of coloured particles and the barrier, and the barrier is positioned relative to the viewing zone such that coloured particles accumulate in the viewing zone following the release of the coloured particles toward and into the viewing zone after cleavage of the polymers by protease activity present in the wound fluid. Thus, the barrier prevents released coloured particles from travelling beyond the viewing zone. For instance, the barrier may comprise, consist essentially of or consist of a porous material wherein the pores are of a size that allows fluid and molecules with a molecular weight below a cut-off value to pass through the barrier but which are not large enough to allow the coloured particles to travel past the barrier. The skilled person is well able to select suitable materials with an appropriate molecular weight cut-off dependent on the coloured particles employed. Alternatively, for embodiments in which the solid support comprises, consists essentially of or consists of a chromatographic medium (such as a nitrocellulose membrane) or other porous membrane, the barrier may comprise a region of the solid support itself wherein the region comprises pores with a pore size smaller than the diameter of the coloured particles (whilst still allowing the passage of fluid). Such a region may be created, for instance, by crushing or otherwise compressing this region of the matrix. The compression is to a degree sufficient to decrease the pore size such that it is smaller than the diameter of the coloured particles. Thus, the coloured particles cannot pass through the crushed/compressed region (barrier) and therefore accumulate in the viewing zone following the release of the coloured particles toward and into the viewing zone after cleavage of the polymers by protease activity present in the wound fluid.

In specific embodiments, the (optionally cross-linked) protease-sensitive polymer comprises, consists essentially of or consists of collagen, optionally forming a collagen plaque. In preferred embodiments, the collagen is fully, substantially or partially denatured prior to use in the invention. Where this has occurred by partial hydrolysis of the collagen, it is termed "gelatin" as would be well-known to the person skilled in the art. Thus, in these preferred embodiments, the polymer comprises, consists essentially of or consists of gelatin. In some embodiments, the polymer comprises, consists essentially of or consists of elastin.

In particular embodiments, the sample application zone and reaction zone at least partially overlap. Thus, wound fluid can be added directly to the reaction zone in some embodiments. This is at an upstream portion thereof to prevent transport of wound fluid through the product in the absence of protease activity.

Thus, the disclosure also provides a product for monitoring the condition of a wound comprising, consisting essentially of or consisting of:
a. a sample application zone to which wound fluid is added;
b. a reaction zone downstream of the sample application zone comprising, consisting essentially of or consisting of coloured (preferably polystyrene) microparticles entrapped within (optionally cross-linked) gelatin; and
c. a viewing zone downstream of the sample application zone and reaction zone;
wherein the sample application zone transmits fluid towards the viewing zone and wherein cleavage of the gelatin by protease activity present in the wound fluid results in carriage of the coloured (polystyrene) microparticles with the wound fluid to the viewing zone thereby providing a visual indication of protease activity in the first viewing zone.

The disclosure also provides a product for monitoring the condition of a wound comprising, consisting essentially of or consisting of:
a. a sample application zone to which wound fluid is added;
b. a reaction zone downstream of the sample application zone comprising, consisting essentially of or consisting of (optionally cross-linked) gelatin;
c. a zone comprising, consisting essentially of or consisting of coloured (preferably polystyrene) microparticles downstream of the reaction zone;
d. a viewing zone downstream of the sample application zone, reaction zone and zone comprising, consisting essentially of or consisting of coloured polystyrene microparticles;
wherein the sample application zone transmits fluid towards the viewing zone and wherein cleavage of the gelatin by protease activity present in the wound fluid results in carriage of the coloured (polystyrene) microparticles with the wound fluid to the viewing zone thereby providing a visual indication of protease activity in the first viewing zone.

All of the aforementioned products and embodiments may further comprise a housing to contain the product. Where a housing is provided it may comprise, consist essentially of or consist of one or more, optionally two, viewing windows for viewing the coloured particles. These viewing windows may be positioned along the housing such that, prior to exposure to wound fluid and in the absence of protease activity, the coloured particles are visible in a first viewing window but are not visible in a second viewing window. The viewing windows served to define the viewing zones of the product. Thus, for those embodiments comprising one or more viewing zones, the one or more viewing windows may be aligned such that the viewing zones are visible through the viewing windows.

In some embodiments, prior to exposure to wound fluid, the protease-sensitive polymers, coloured particles and optionally one or more cross-linking agents may be mixed together and applied as a mixture to the reaction zone (which may overlap with the sample application zone as described elsewhere herein). Still prior to exposure to wound fluid, the mixture may be dried or cured (e.g. using UV light) to promote cross-link formation if one or more cross-linking agents are present and/or to fix in the reaction zone the (optionally cross-linked) protease-sensitive polymers in which the coloured particles are thus entrapped. This procedure may be done at the point of care. Therefore, the disclosure also provides a kit of parts comprising:
(i) a product comprising a sample application zone, reaction zone (absent of (optionally cross-linked) protease-sensitive polymers and coloured particles) and a viewing zone as defined herein;
(ii) protease-sensitive polymers;
(iii) coloured particles; and
(iv) optionally one or more cross-linking agents (preferably methacrylate, methacrylic anhydride or a derivative thereof).

In some embodiments, the protease-sensitive polymers and coloured particles are provided as a pre-formed mixture.

The product of the kit of parts disclosed herein (see feature (i) above) may further comprise one or more of the additional features described herein such as a housing, a second viewing zone and/or a barrier. These features are not repeated here simply for conciseness.

In alternative embodiments, prior to exposure to wound fluid, the protease-sensitive polymers and optionally one or more cross-linking agents may be mixed together and applied as a mixture to the reaction zone (which may overlap with the sample application zone as described elsewhere herein). The coloured particles are applied to a distinct zone positioned between the reaction zone and the viewing zone. Still prior to exposure to wound fluid, the mixture may be dried or cured (e.g. using UV light) to promote cross-link formation if one or more cross-linking agents are present and/or to fix in the reaction zone the (optionally cross-linked) protease-sensitive polymers. This procedure may be done at the point of care. Therefore, the disclosure also provides a kit of parts comprising:
(i) a product comprising a sample application zone, reaction zone (absent of (optionally cross-linked) protease-sensitive polymers), a viewing zone and a zone positioned between the reaction zone and viewing zone to receive the coloured particles as defined herein;
(ii) protease-sensitive polymers;
(iii) coloured particles; and
(iv) optionally one or more cross-linking agents (preferably methacrylate, methacrylic anhydride or a derivative thereof).

In some embodiments, the protease-sensitive polymers and coloured particles are provided as a pre-formed mixture.

The product of the disclosed kit of parts (see feature (i) above) may further comprise one or more of the additional features described herein such as a housing, a second viewing zone and/or a barrier. These features are not repeated here simply for conciseness.

In particular embodiments, the matrix comprises, consists essentially of or consists of a chromatographic medium. In some embodiments, the matrix comprise, consist essentially of or consist of one or more capillary flow channels along/through which wound fluid can travel. In addition, the matrix may be etched such that wound fluid once applied to the sample application zone (or similar as described herein) is concentrated as it travels along/through the matrix. Thus, advantageously, the quantity of wound fluid needed to detect protease activity using the product is reduced.

Alternatively, the portions of the product that do not comprise, consist essentially of or consist of the reaction zone and/or viewing zone(s) may be masked so that any coloured particles are not visible to the end user. This helps to simplify the signals to be interpreted.

As defined in the claims the invention also provides a method of monitoring the condition of a wound comprising:
i. applying a sample of wound fluid to the sample application zone of a product comprising, consisting essentially of or consisting of:
   a. a sample application zone to which wound fluid is added;
   b. a reaction zone downstream of the sample application zone comprising, consisting essentially of or consisting of (optionally cross-linked) protease-sensitive polymers;
   c. a zone comprising, consisting essentially of or consisting of coloured particles downstream of the sample application zone; and
   d. a viewing zone downstream of the sample application zone, reaction zone and zone comprising, consisting essentially of or consisting of coloured particles;
   wherein the sample application zone transmits fluid towards the viewing zone (which prior to exposure to wound fluid and in the absence of protease activity, does not contain coloured particles) and wherein cleavage of the polymers by protease activity present in the wound fluid results in carriage of the coloured particles with the wound fluid to the viewing zone thereby providing a visual indication of protease activity in the viewing zone; wherein:
   (i) the coloured particles are entrapped within the polymers and cleavage of the polymers by protease activity present in the wound fluid results in release of the coloured particles to the viewing zone thereby providing a visual indication of protease activity in the wound fluid via the viewing zone; or
   (ii) the reaction zone forms a barrier that prevents wound fluid reaching the viewing zone and wherein cleavage of the polymers by protease activity present in the wound fluid, optionally above a threshold level, disrupts the barrier and results in carriage of the coloured particles to the viewing zone thereby providing a visual indication of protease activity in the wound fluid via the viewing zone; and
ii. detecting the visual indication of protease activity provided by the coloured particles in the viewing zone.

The coloured particles may, in some embodiments, be entrapped within the protease-sensitive polymers in the reaction zone prior to exposure to wound fluid. Thus, the disclosure also provides a method of monitoring the condition of a wound comprising, consisting essentially of or consisting of:
i. applying a sample of wound fluid to the sample application zone of a product comprising, consisting essentially of or consisting of:
   a. a sample application zone to which wound fluid is added;
   b. a reaction zone downstream of the sample application zone comprising, consisting essentially of or consisting of coloured particles entrapped within (optionally cross-linked) protease-sensitive polymers; and
   c. a viewing zone downstream of the sample application zone and reaction zone;
   wherein the sample application zone transmits fluid towards the viewing zone (which prior to exposure to wound fluid and in the absence of protease activity, does not contain coloured particles) and wherein cleavage of the polymers by protease activity present in the wound fluid results in carriage of the coloured particles with the wound fluid to the viewing zone thereby providing a visual indication of protease activity in the first viewing zone; and
ii. detecting the visual indication of protease activity provided by the coloured particles in the viewing zone.

The product used in these methods may contain one or more of the features described above. These features are not repeated here simply for conciseness.

In particular embodiments of the method, the coloured particles measured in the viewing zone provide a quantitation of the protease activity in the wound fluid. In some embodiments, the product employed in the methods comprises two viewing zones (as described elsewhere herein) and loss of coloured particles in the second viewing zone positioned above (i.e. in vertical alignment with) the zone comprising, consisting essentially of or consisting of coloured particles (in the absence of wound fluid and protease activity) is compared and/or quantitated with gain in coloured particles in the viewing zone downstream of the zone comprising, consisting essentially of or consisting of coloured particles following exposure to wound fluid and protease activity. In preferred embodiments, the change in colour in each viewing zone is visible to the naked eye. Alternatively, or in addition to, observance via the naked eye, the change in colour can be detected and/or quantified using a spectrophotometer or other such equipment suitable for this purpose, which are well-known to the skilled person.

In some embodiments, the method further comprises applying additional liquid to the product to assist with fluid flow of the wound fluid. This may be termed a "chase fluid". The additional liquid may comprise, consist essentially of or consist of a buffer. The buffer may be matched to the properties of the wound fluid and/or other materials employed in the method. For example, a "chase fluid" may be employed in order to carry the wound fluid through the pores of a test strip. Use of additional liquid can improve transmission of released coloured particles along/through the matrix. The additional liquid may comprise, consist essentially of or consist of a surfactant to prevent unwanted adhesion of the coloured particles to the matrix.

In another aspect of the invention, the methods described herein are repeated at intervals in order to facilitate longitudinal monitoring of the condition of the wound by repeated sampling and analysis of the wound fluid. Said intervals may be every 1, 2, 3, 4, 5 or 6 days, weekly or monthly or a combination thereof. The aggregation of data pertaining to the condition of the wound over time better enables the clinician to understand the progress of the condition of the wound and/or efficacy of treatment(s). For instance, longitudinal monitoring of the wound fluid as described may indicate to the clinician, in a more rapid and/or quantitative fashion than current procedures, that the condition of the wound is deteriorating over time and thus the present treatment is ineffective. Consequently, the clinician can more rapidly select alternative treatments in order to promote healing of the wound. Alternatively, the data may indicate to the clinician that further tests of the wound fluid and/or wound environment are needed.

In a further embodiment, the absence of excess protease activity indicates that any existing treatment of the wound should be continued (i.e. should not be altered).

In certain embodiments according to all aspects of the invention, the wound is a chronic wound. A "chronic wound" should be understood to be a wound in which the normal process of wound healing is disrupted at one or more points in the phases of wound healing. For instance, a wound stuck in a particular phase of healing such as inflammation or proliferation. A chronic wound may be characterized by a raised, hyperproliferative, yet non-advancing wound edge and/or a local wound environment, rich in inflammatory products, and proinflammatory cytokines comprising, consisting essentially of or consisting of an imbalanced enzymatic milieu consisting of an excess of matrix metalloproteases and a reduction in their inhibitors resulting in the destruction of the extracellular matrix. Common chronic wounds include diabetic ulcers, vascular ulcers and pressure ulcers.

In certain embodiments according to all aspects of the invention, the subject from which wound fluid is obtained is an animal. In particular embodiments, the animal is a human.

In particular embodiments, a product as described herein is used in a method as described herein.

### DESCRIPTION OF THE FIGURES

Figure 1A is a schematic illustrating one example of the invention wherein a product as described herein is placed in contact with a wound underneath a wound dressing.
Figure 1B is a schematic illustrating the generation of a visual indication by a product as described herein following absorption of wound fluid and cleavage of the cross-linked protease-sensitive polymers by protease activity present in the wound fluid.
Figure 2 is a schematic illustrating a lateral-flow based embodiment of a product as described herein.
Figure 3 is a schematic illustrating a further lateral-flow based embodiment of a product as described herein.
Figure 4 shows the NMR spectra of unmodified gelatin prior to methacrylate modification.
Figure 5 shows the NMR spectra of fully methacrylate modified gelatin (GELMA).
Figure 6 shows a top-view (A) and exploded side-view (B) of an exemplary product according to the invention, preferably for *in situ* use in a wound.
Figure 7 demonstrates a product as described herein in use (time = 0hr).
Figure 8 shows the results using the product shown in figure 7 in the presence and absence of papain after 24, 48 and 72hr.
Figure 9 demonstrates a further product as described herein in use (time = 0hr).
Figure 10 shows the results using the product shown in figure 9 in the presence and absence of matrix metalloproteinase 9 (MMP9) and human neutrophil elastase (HNE) after 24 and 48hr.
Figure 11 shows the results using the product shown in figure 9 in the presence and absence of matrix metalloproteinase 9 (MMP9) and human neutrophil elastase (HNE) after 120 and 144hr.

### DETAILED DESCRIPTION OF THE INVENTION

The invention will now be described, without limitation but solely to aid understanding of the invention, by reference to the Figures.

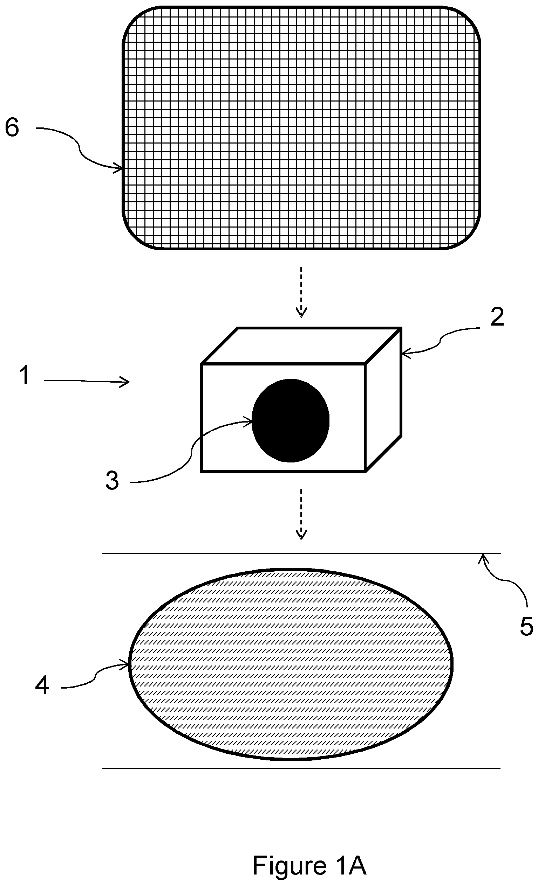

A product (1) as described herein is shown schematically in Figure 1A. The product (1) comprises, consists essentially of or consists of a matrix (2), preferably biologically inert for *in situ* applications, which can absorb wound fluid and coloured particles entrapped within cross-linked and protease-sensitive polymers (3), in this case coloured polystyrene microspheres (PSM) entrapped within cross-linked gelatin, on or in the matrix (2). The coloured particles entrapped within the cross-linked gelatin (3) may be dried into, or conjugated to, the matrix (2) and, in the case of suitably coloured PSM, make the whole or a substantial part of the matrix appear black in colour, forming a test unit or reaction zone. The coloured particles entrapped within the cross-linked gelatin (3) are used to measure protease (gelatinase) activity in fluid released by a wound. The cross-linked gelatin (3) is degraded by gelatinases present (optionally at or above a threshold concentration) in the wound fluid.

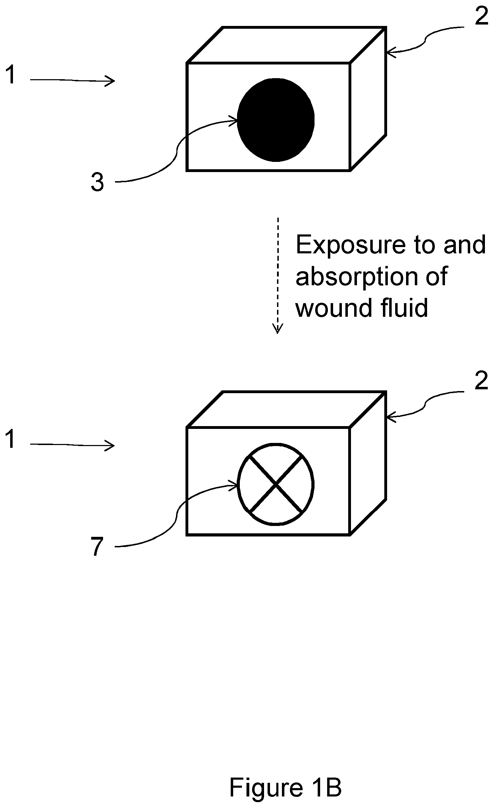

When the product (1) is placed in contact with the wound (4), which may be a chronic wound, on a subject (5) underneath a wound dressing (6), the matrix (2) absorbs wound fluid. If the wound fluid comprises sufficient gelatinase activity, the cross-linked gelatin (3) on or in the matrix (2) is degraded into fragments, in particular if the gelatinase activity is present at or above a threshold level. Once degraded, the PSM are no longer entrapped and are free to disperse through/along the matrix. In this case, as shown in Figure 1B, dispersal of the PSM through/along the matrix removes the black colouration that was attributed to the reaction zone of the matrix by the PSM prior to exposure to wound fluid and reveals a visual symbol printed on the product such as a cross (7). The revelation of the visual symbol indicates the presence of aberrant protease activity in the wound.

While the cross (7) provides a positive test result, and is therefore advantageous, it is not essential. Instead dissipation of the colouration can be used as an outcome of the test without revealing a further symbol.

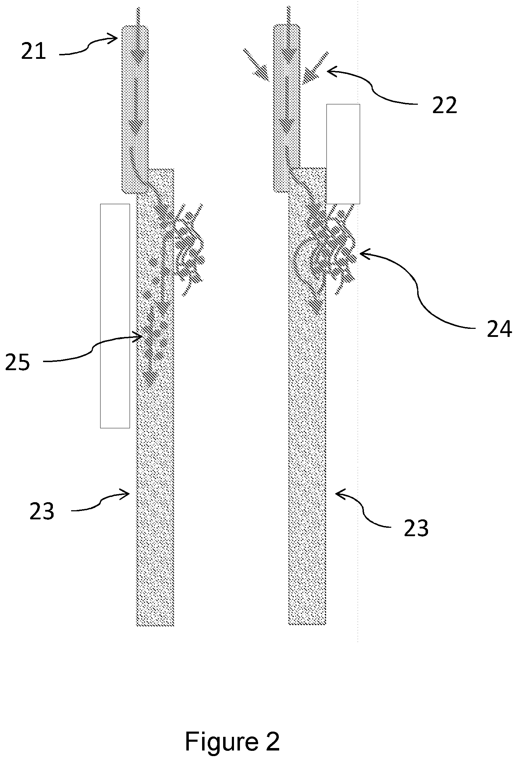

Figure 2 is a schematic illustrating a lateral-flow based embodiment of a product as described herein. A sample application zone is shown (21) to which is applied wound fluid (represented by the straight grey arrows (22)). In this particular case, the sample application zone is a flexible wick which acts as a swab for collecting the wound fluid (22). This wick (21) is fluidly connectable with solid support (23) which in this case is a porous test strip in which fluids flow by capillary action (analogous to that of a lateral flow immunoassay). Methacrylate cross-linked gelatin (GELMA) in which coloured PSM are entrapped form a reaction zone (24) on the solid support. Wound fluid is transmitted to the reaction zone (24) via capillary action. Protease activity present in the wound fluid cleaves the GELMA and releases the PSM. The shear stress of the moving fluid entrains the released PSM and these are carried forward with the fluid into a viewing zone (25) thereby providing a visual indication of protease activity in the wound fluid.

In alternative embodiments, the gelatin may be cross-linked using glutaraldehyde or a derivative thereof instead of methacrylate. Prior to exposure to wound fluid, the gelatin, coloured PSM and gluteraldehyde may be mixed together and applied as a mixture to form the reaction zone (24) on the solid support. Still prior to exposure to wound fluid, the mixture may be dried to promote cross-link formation and to fix the gluteraldehyde cross-linked gelatin, in which the coloured PSM are thus entrapped, to the solid support (23) thereby forming the reaction zone (24).

In further embodiments, the product, comprising at least the solid support (23) and optionally also the wick (21) may be housed in a casing with a viewing window downstream (in the direction of fluid travel) of the reaction zone (24). In such embodiments, the viewing window defines the viewing zone (25). Thus, PSM released following cleavage of the GELMA or gluteraldehyde cross-linked gelatin by protease activity in the wound fluid can be observed in the viewing window. Optionally, a second viewing window can be positioned above the reaction zone (24). Thus, loss of colour can be observed through this additional viewing window should PSM be released due to protease activity present in the wound fluid.

In yet further embodiments, the viewing zone (25) may comprise, consist essentially of or consist of capture molecules capable of specifically binding the PSM. Thus, released PSM are arrested in position and concentrated to assist observation and potentially quantitation. The capture molecules may comprise, consist essentially of or consist of antibodies which specifically recognise antigens bound onto the surface of the PSM or it could comprise, consist essentially of or consist of some other molecular species to which the PSM can be made to bind, including charged molecules to bring about ion-exchange interactions.

In further embodiments, the device can be coupled with a source of matched buffer solution such as phosphate buffered saline pH 7.2 (with surfactant to prevent unwanted adhesion of PSM to the test strip) to act as a "chase fluid" in order to carry the wound fluid through the pores of the test strip and maximise the extraction of PSM from the matrix (when proteolysis has started) and efficiently transport them to the viewing zone (24).

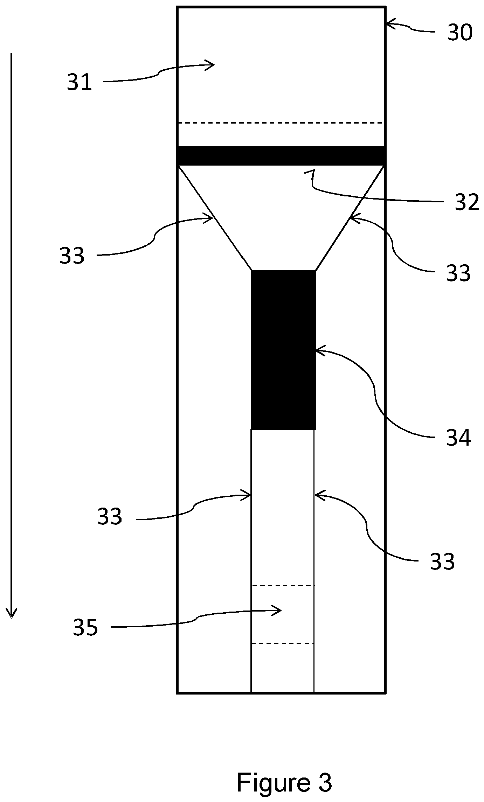

Figure 3 is a schematic illustrating a further lateral-flow based embodiment of a product as described herein.

The product comprises, consists essentially of or consists of a solid support (30) comprising, consisting essentially of or consisting of a sample application zone comprising, consisting essentially of or consisting of an absorbent material (31), a barrier (reaction zone) formed from protease-sensitive polymer molecules, in this case gelatin (32), a zone comprising, consisting essentially of or consisting of coloured particles (34), in this case Monastral blue dye molecules (MBDM), and a viewing zone (35). Optionally, the product also comprises etchings (33) to guide and concentrate any fluid along/through the matrix.

In operation, a sample of wound fluid is added to the sample application zone (31) and travels via capillary action in the direction indicated by the large arrow (shown in Figure 3 parallel to the product schematic) until it reaches the gelatin barrier (32). In the absence of protease activity in the wound fluid, the gelatin plug remains intact and prevents the wound fluid from passing beyond it. Thus, the MBDM remain dried to the matrix and are not transmitted toward and into the viewing zone. Consequently, no visual indication of protease activity is provided in the viewing zone. However, in the presence of protease (gelatinase) activity in the wound fluid, the gelatin plug is cleaved and wound fluid can now travel via capillary action toward and into the viewing zone. In so doing, the wound fluid carries with it MBDM into the viewing zone thereby providing a visual indication of protease activity in the wound fluid.

In further embodiments, the solid support (30) may be housed in a casing with a viewing window downstream (in the direction of fluid travel) of the zone comprising, consisting essentially of or consisting of coloured particles (34), and which encompasses, and may define, the viewing zone (35). Thus, coloured particles (e.g. MBDM) released following cleavage and disruption of the gelatin barrier by protease activity in the wound fluid can be observed in the viewing window. Optionally, a second viewing window can be positioned above the zone comprising, consisting essentially of or consisting of coloured particles (34). Thus, loss of colour can be observed through this additional viewing window should MBDM be released due to protease activity present in the wound fluid.

### EXPERIMENTAL SECTION

### Example 1: Production of GELMA from gelatin and methacrylate

Gelatin was modified by end-capping with methacrylic anhydride, affording methacrylamide terminated gelatin, termed herein "GELMA" (general scheme shown below).

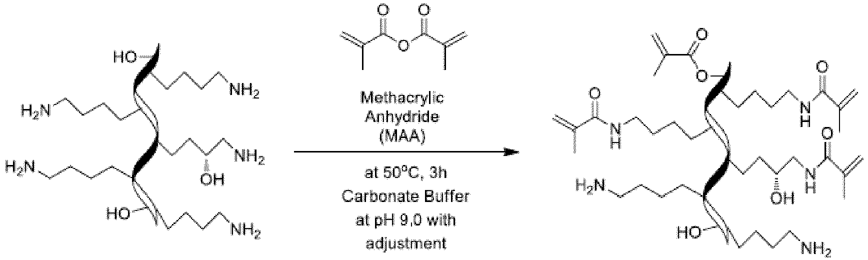

There are many references in the literature which use a variety of different methods and conditions to produce this material but the method used by Bae Hoon Lee et.al (RSC adv., 2015, 5, 106094*)* was adopted for this purpose, as follows.

Type A gelatin, obtained by acid treatment at pH1-2 (an isoelectric point of pH7-9) was used. In order to get efficient substitution (endcapping) a high excess of methacrylic anhydride was needed. Since the by-product from the reaction is methacrylic acid the pH of the reaction was gradually lowered to below the iso-electric point range, resulting in reduced endcapping. To circumvent this, Bae Hoon Lee *et.al.* used a carbonate buffer to keep the reaction mixture between pH 7 & 9 and the alternate addition of methacrylic anhydride and sodium hydroxide for pH control. This method allowed for efficient endcapping with the least amount of side reactions (termination of hydroxyl groups with methacrylic ester). It also reduced the amount of reagents used and minimised the amount of inorganic salts to be removed in the purification step. The reaction product was purified by tangential flow filtration (TFF), although simple dialysis can also be used.

### Detailed method

Gelatin type A [175 bloom] (10g) and 100ml of carbonate buffer 0.1M (100ml) [3.18g NaCO3, 5.86g NaHCO3] were mixed in a round bottomed flask fitted with an overhead stirrer, temperature probe and pH meter. The mixture was heated to 60°C to dissolve the solid. The reaction temperature was then reduced to 50°C and the pH checked and adjusted to 9.0.

Methacrylic acid (167µl) was added dropwise to this solution causing the pH to drop to -8.5 and the reaction mixture was stirred for 25mins. After this, the pH was then adjusted back to pH 9.0 with 20% NaOH solution and stirred for a further 5 mins.

Further rounds of methacrylic acid and pH adjustment were repeated a further 5 times until a total of 1 mL of methacrylic acid had been added during a total of 3 hrs reaction time.

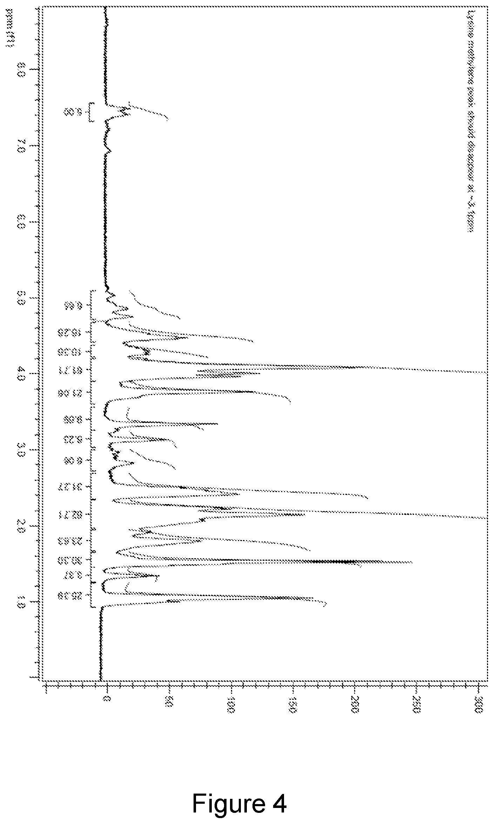

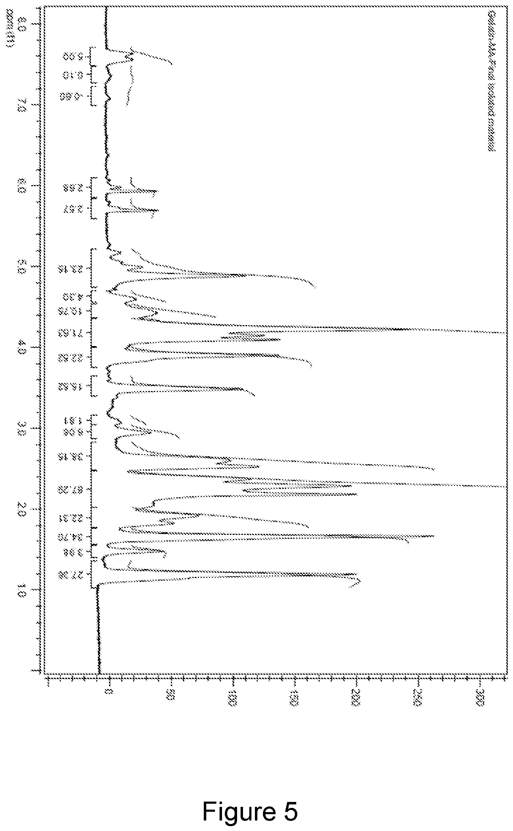

Finally, the pH was adjusted to 7.4 with 50% acetic acid. The resultant mixture was filtered through 0.2 micron PTFE filter membrane into a tangential flow filtration reservoir. The mixture was then purified by dia-filtering against water (water for irrigation) using a 3kD midi-Kros MPEs TFF membrane with a transmembrane pressure of ∼15. A total of 6L was dia-filtered to obtain a permeate conductivity <10 µS. The resulting pale yellow solution was transferred to a Florentine flask and freeze dried for 2 days. This afforded a white polystyrene-like material 8.7g, ready for use in preparing a product as described herein. The degree of methacrylate substitution in the product was determined by proton NMR. The results are shown in Figures 4 and 5. Note the loss or depletion of peaks from Figure 4 (e.g. the lysine methylene peak at ∼3ppm) and the appearance of new peaks in Figure 5 (e.g. at 4.8ppm, 5.7 and 5.9) indicating the incorporation of methacrylate groups in the GELMA product. These results confirm the reaction to have progressed well with the loss of peak at ∼2.95-3.1 ppm.

**Elemental Analysis. (CHN and Na)**

| Element | Carbon | Hydrogen | Nitrogen | Sodium |
|---|---|---|---|---|
| % Found | 45.88 | 6.42 | 16.21 | 0.71 |

These data show there is still some sodium present but this did not cause a problem.

### Discussion

This method had been followed twice on a 2g pilot scale. One reaction product was purified by dialysis (12-14KD cut off), the other by TFF (3KDa cut off). Both materials performed equally well for manufacture of a product as described herein. Due to the ease and efficiency of the TFF purification it was decided to use this method to purify the larger 10g batch. The method was reproducible, simple and efficient.

### Conclusion

The method used to generate gelatin methacrylate (GELMA) is fit for this purpose. If required, the skilled person could further optimise the formulations by known methods and approaches, such as constant pH control with automated base addition. Scale-up for commercial application can be readily achieved by known methods and procedures.

### Example 2: Manufacture of a product according to the invention

The manufacturing process includes all or some of the following operations, the details of which can be optimised in accordance with the needs of user and the constraints of the manufacturing plant.

| Step | Task |
|---|---|
| 1 | Coloured reagent preparation |
| 2 | Absorbent material |
| 3 | Permanent "+" indicator printing |
| 4 | Deposition of coloured reagent |
| 5 | Fixation of coloured reagent (achieved by cooling to solidify, and exposure to UV) |
| 6 | Drying of the coloured reagent |
| 7 | Application of top cover |
| 8 | Conversion and packaging |
| 9 | Sterilisation |

A particular non-limiting format, which has been found to be useful as a practical and readily manufactured product is as follows:
- A disk of absorbent medical material made from a medical foam measuring 2.5mm in depth and with a diameter of 15mm diameter.
- A "+" symbol is printed with permanent ink on the top side of the disk in a central position. The printed "+" measures 4mm across.
- The "+" is covered (therefore hidden from view) by a coloured reagent comprising, consisting essentially of or consisting of cross-linked methacrylate modified gelatin (GELMA)
- The product has an elastic, thin, transparent covering material adhered to the top surface. The preferred covering is a medical grade polyurethane.
- The individual 15mm disks are packaged as single unit, and sterilised.

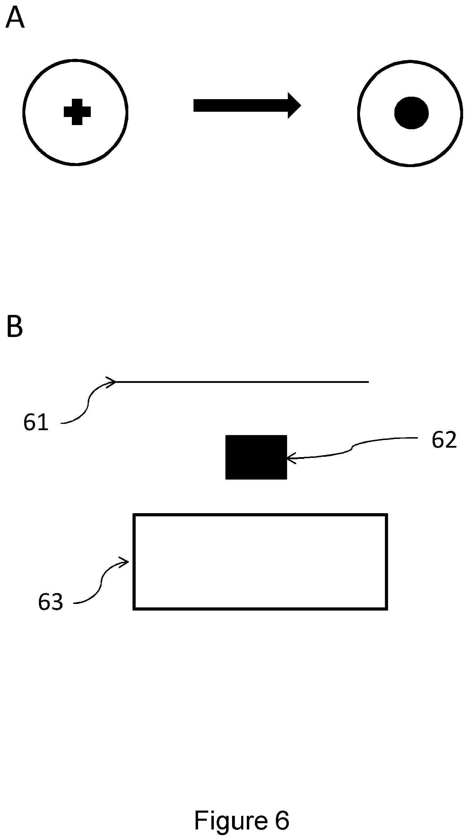

A schematic of such a product is shown in Figure 6. Figure 6A shows a top-view of the product. The disc marked with a permanent cross is covered with and obscured by the application of the coloured reagent comprising, consisting essentially of or consisting of cross-linked methacrylate modified gelatin (GELMA). Figure 6B shows an exploded sideview of the product comprising, consisting essentially of or consisting of a polyurethane film (61), the coloured reagent comprising, consisting essentially of or consisting of cross-linked methacrylate modified gelatin (GELMA) (62) and an absorbent matrix (63).

### Example 3: Manufacture - Wet deposition of the GELMA reagent and assembly

Stock materials required for product:
- Absorbent material roll stock
- Polyurethane roll stock (with adhesive already applied)
- Modified gelatin (GELMA)
- Dyed polystyrene microparticles
- Permanent ink
- Moisture impermeable packaging (likely to be laminated foil)

The following steps constitute the manufacturing process:

| Step | Task | Description |
|---|---|---|
| 1 | Coloured reagent preparation | Modified gelatin GELMA is prepared, simply by dissolving into water at elevated temperature. Coloured polystyrene microparticles are added to the mix. The mix is kept at 30°C or above to prevent solidification. |
| 2 | Absorbent material | Roll stock of absorbent material unwound onto assembly equipment |
| 3 | Permanent "+" mark printing | A printing station applies the "+" symbol. The ink needs to be fixed in place before the application of the coloured reagent. |
| | | - By heat drying for solvent based ink |
| | | - By UV for UV activated ink |
| 4 | Deposition of coloured reagent | A fixed volume (5 microlitres) of the GELMA and polystyrene microparticle mix is applied to cover the printed permanent "+" symbol. |
| | | - The storage vessel and deposition lines/nozzle maintained at a temperature of 30°C or above to prevent solidification of the coloured reagent and therefore blocking of the equipment. This can be achieved by keeping the temperature of the room at a minimum of 30°C |
| 5 | Fixation of coloured reagent (achieved by cooling to solidify, and exposure to UV) | The coloured reagent stays in place after deposition due to the viscosity of the solution. |
| | | The deposited coloured reagent is then fixed with high intensity UV. Exposure time is 15-30 seconds. |
| 6 | Drying of the coloured reagent | Due to the small volume of the deposited reagent, the drying is a quick process, accelerated by passing through a heated air tunnel. |
| 7 | Application of top cover | A medical polyurethane (PU) cover is applied to the absorbent product. |
| | | - This can be achieved by the use of a pre-coated PU, or |
| | | - By applying adhesive to the absorbent material then apply an un-coated PU |
| 8 | Conversion and packaging | 15mm disks are cut from the finished laminated roll-stock, and individually packaged. |
| 9 | Sterilisation | The final product will be sterilised. |
| | | - This can be achieved by gamma or e-beam irradiation, or by ethylene oxide or dry heat (e.g. in sealed pouches in an autoclave at 121°C) |

| | | |
|---|---|---|
| NOTES: Step 4: - The coloured reagent needs to be kept at an elevated temperature during storage and deposition. This is to prevent the reagent from solidifying. - The volume to be deposited is very small (5 microlitres per disk). The viscosity of the warmed modified gelatin is not high, although it is higher than water. If necessary, the viscosity can be enhanced by adding a thickening agent (e.g. carboxy methyl cellulose or just more unmodified gelatin). - The coloured reagent should not be absorbed into the absorbent material, otherwise the indicator matrix will not become uniformly cross-linked and the depth of the matrix it forms will be compromised. This has an effect on the choice of the absorbent material and/or the properties of the pre-cross-linked GELMA reagent. - The deposition of the coloured reagent needs to be aligned with the printed "+" symbol. The coloured reagent can be circular in presentation, as long as it covers (obscures) all of the pre-printed "+" symbol. Registration is therefore important. Step 5: - A UV lamp is used to fix the coloured reagent in place. The exposure time is 15- 30 seconds, depending on the power output of the lamp. So far, lamps which deliver ∼300nm and ∼380nm UV lamps, at energy levels ranging from 1-100mW/cm² have been found to be effective. Step 6: The fixed coloured reagent is dried before packaging. A hot air tunnel may be used to dry the coloured, matrix protease. Other options may be considered. | | |

### Problems encountered and overcome by the exemplified product for in situ use

Problems encountered:
1) Modifying the properties of dried gelatin to make it more suitable for tests in which the matrix is exposed to wound fluid for extended periods. Gelatin (denatured collagen) in its normal state (as purchased or derived from collagen) can be dried to form suitable matrix structures but it is gradually dissolved by aqueous fluids containing dissolved proteins, such as wound fluids at typical wound temperatures, even in the absence of protease. On the other hand, excessively modified gelatin can become too resistant to protease cleavage. Although un-modified gelatin can work under certain conditions, the cross-linked version is more robust and capable of working over longer time periods.
2) In manufacturing, the deposited gelatin solution must be very quickly fixed in place, such that it becomes firmly adhered to the carrier material, otherwise it will become smeared or dislodged. Alternatively very slow, uneconomic processing would have to be used.
3) Dye-modified gelatin may not form a sufficiently intense colour when deposited on a suitable carrier surface in a depth sufficiently thin to allow destruction by clinically relevant levels of protease.
4) The simplicity of operation desired for this application does not allow for complex processes such as immunoassays or other procedures that require more than a single operational step.
5) Because the device must be in physical contact with the wound, it is not possible to use reactive ingredients which have not been approved for use in wounds or do not have a history of use in wounds.
6) Some aspects of operation planned for the device require that it can be left in-situ on the surface of the wound, under a dressing for at least one day and potentially for several days.

| Problem to be overcome | How the problem is overcome | Comments |
|---|---|---|
| 1 | Controlled modification of lysine epsilon amino groups in the gelatin molecules with polymerisable functional groups such as methacrylate (to form GELMA), which allows subsequent controlled cross-linking. Alternatively, direct cross-linkers such as glutaraldehyde may be used if carefully controlled. | This approach prevents premature solubilisation of dried gelatin by simple dissolution in aqueous fluid. It requires protein chain cleavage before the matrix can be dissolved. |
| 2 | During manufacture, with methacrylate modification, the GELMA cross-linking can be delayed until the liquid matrix is dispensed onto the carrier, whereupon irradiation with UV light causes an immediate, robust cross-linking to occur. | This is highly compatible with production line processing and yields an ideal indicator matrix |
| 3 | Instead of using soluble dyes that associate (reversibly) with the gelatin molecules, or are covalently attached to the gelatin, coloured polystyrene microparticles (CPSM) are mixed with the GELMA. These become firmly enmeshed in the cross-linked gelatin molecular network, but readily escape when the gelatin molecules are cleaved by the proteases. | Very dense coloration of the matrix is achieved by the use of CPSM. The process of CPSM escape and consequent decolourisation happens easily and without agitation of fluid flow, even in completely static conditions. |
| 4 | CPSM entrapment in the cross-linked GELMA molecular network provides a coloured matrix which then decolourises simply by dispersal of the particles. As this requires no intervention or sequential processing, the test takes place without any user involvement or any accessory devices, actions or controlled interactions | This is a true single-step process and is unlike any other protease test encountered by the inventors. |
| 5 | The only chemical entities which come into contact with the wound are gelatin, gelatin fragments and methacrylate-amino-acid compounds. Of course, gelatin is a natural substance with a long history of safe use in wounds. Methacrylate componds have long been in use in wounds as ingredients of polymeric hydrogels. Any dye molecules are entrapped within the CPSM, and polystyrene particles are non-toxic substances, varieties of which have been approved for use in wounds. The carrier material is also selected from materials already approved for use in wounds. | All of these ingredients are known to be basically biocompatible. |
| 6 | Because of the basic biocompatibility of the ingredients, the extreme simplicity of the structure and the assay process, together with the very low physical profile (no more than 2.5mm thick, the test device can be placed on the wound under any cover dressing without mutual interference. | The functioning of the protease activity test is not compromised under the dressing. It can be left in place for less than a day or for many days. |

The product can be in the form of a very thin structure with little or no fluid collection capability. It can be presented as an *in situ* test unit built into a non-woven carrier which is simply "wettable" by wound fluid. It can sit on the wound surface in this mode, where it can function simply as a low-cost, supremely easy-to-use protease activity indicator, under a dressing if required for variable lengths of time. In its very simplest embodiment, it could even be used without a carrier, presented just as a coloured, cross-linked piece of gelatin film, although this would be less easy to handle and observe. Alternatively, the indicator matrix can be part of a composite unit with dimensions of 15mm X 2.5mm, in which there is a transparent cover, a non-woven carrier layer and a foam sample-collector layer.

### Example 4: Demonstration of a product according to the invention in use based on methacrylate cross-linked gelatin

### Materials used

GELMA: modified gelatin with methacrylic anhydride. (Methacrylic anhydride sourced from Sigma-Aldrich (product number 276685). Gelatin sourced from Sigma Aldrich, Porcine Type A, 300 bloom (product number G2500))

Photo initiator "Daracure", 2-hydroxy-4'-(2hydroxyethoxy)-2-methylpropiophenone sourced from Sigma Aldrich (product number 410896)

Polybead® Polystyrene Blur Dyed Microsphere (0.5 micron diameter, 2.5% solids) sourced from Polysciences, Inc. (product number 15709)

Polyurethane film sourced from Coveris Advanced Coatings (Wrexham, UK), (Inspire 2331)

Polyurethane foam sourced from foam Freudenberg, formerly Polymer Health Technologies; (Ebbw Vale, UK), (free sample)

### Method

A 0.5% w/v photo initiator (PI) solution was prepared by adding 2.5mg of Daracure powder to 500µl 1x concentration phosphate buffered saline (PBS). The mixture was heated to 60°C with periodic vortex mixing until all solids had dissolved. After the photo initiator had been completely dissolved, 50mg of GELMA powder was added to the solution, which was gently mixed to allow the GELMA to dissolve. During this dissolution process, the temperature of the GelMA/PI/PBS solution was reduced to 40°C with periodic vortex mixing. The temperature of the solution was maintained at 40°C to prevent the GELMA solidifying to a gel. Blue polystyrene microspheres were finally added at a ratio of 9 parts GELMA/PI/PBS to 1 part micro particle stock solution. The solution was mixed thoroughly to ensure a homogenous solution. The final concentrations within the mixture were: 0.45% photo initiator, 8.1% GELMA and 0.25% micro particles.

5µl aliquots of the GELMA/microsphere/photo-initiator mix were taken and deposited centrally onto the adhesive surface of a 30µm transparent polyurethane film (Coveris advanced coatings, Inspire 2331) at the rate of one 5µl spot per 10 x 10mm square of film. Each drop was gently agitated to disperse the drop of blue mixture into a 4-5mm diameter blue circle. The polyurethane (PU) film squares were then placed under a broad spectrum UV lamp (Dr. Honle, Germany; power c.100mW/cm²) for 15 seconds to initiate crosslinking and polymerise the GELMA. Finally the polymerised spots on the PU squares were air-dried at ambient temperature for 3 hours.

After the spots had been dried, each PU square (with its own single blue indicator spot) was affixed via the exposed adhesive onto a similarly sized piece of PU foam (5mm thick), to create a set of composite squares. Thus, each square consisted of a layer of PU foam, topped with a piece of thin transparent PU film, held in place by a layer of adhesive with a dry, blue spot of cross-linked GELMA held between the two layers. The blue GELMA spot was positioned with its uppermost surface in contact with the adhesive and its lower surface in direct contact with the PU foam. Using a permanent marker pen, a 4mm (height and width) blue cross was drawn on the upper most surface of each composite square (on the top surface of the transparent PU film layer) such that the cross is positioned above the blue GELMA spot so that no part of the drawn cross extended beyond the outer edge of the blue indicator. With this arrangement, the blue cross was visually obscured by the intensely blue spot beneath it. When this had been completed, the squares were ready to be used as indicators of protease activity.

To test the responsiveness of the assembled indicators, three test solutions were prepared, each based on a standard papain activation buffer with the following composition:- 4.22mM I-Cysteine HCL, 3.6mM EDTA, 0.2M NaCI in deionised water, pH 7. Test solution 1 contained 0.1mg/ml of papain (approx. 1000unit/g), test solution 2 contained 0.001 mg/ml of papain and test solution 3 contained 0mg/ml papain. Two of the prepared indicator squares were placed in a petri dish containing solution 1, so that the absorbent PU foam became saturated with the solution. In this situation, the solution was brought into direct contact with the blue spot. Two squares were similarly placed in solution 2 and a further two were placed in solution 3. The squares were photographed before they were placed in contact with the test solutions (i.e. in a dry state) and again when initially wetted with the test solutions, and yet again when 24, 48 and 72 hours at 37°C had elapsed after first contact with the test solutions.

### Results

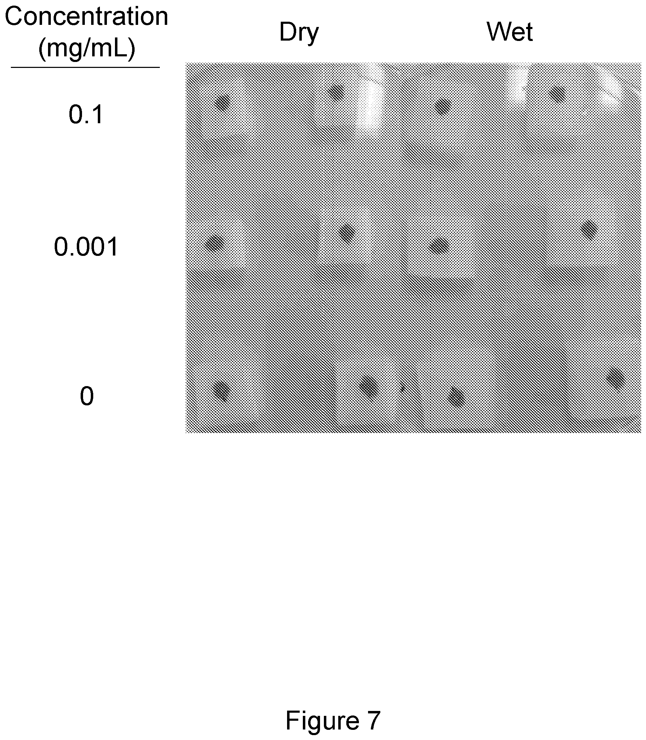

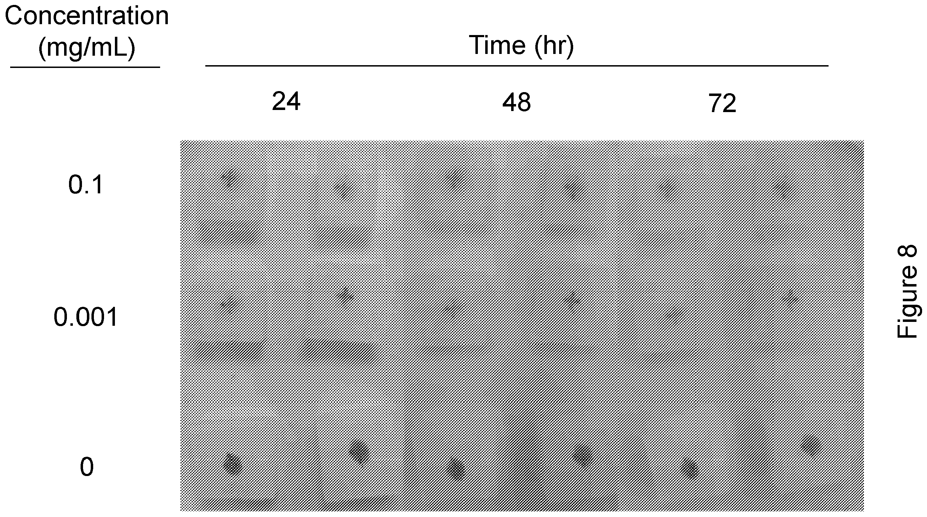

The state of the blue GELMA spots at the pre-determined time points of the experiment are shown in Figures 7 and 8. In the dry state and on first wetting (i.e. time = 0hr) with any of the test solutions it can be seen that the blue GELMA spots are completely intact (Figure 7). In this condition the blue cross has not been exposed on any of the squares. At each of the subsequent time points almost complete digestion of the GELMA was observed in the presence of both concentrations of papain (Figure 8). This is seen by the change in presentation, where the blue coloured spot changes to the blue coloured cross. This is because the gelatin component of the GELMA has been digested by the active protease enzyme, which in turn releases the entrapped and immobilised microspheres. The coloured microspheres then disperse, spreading evenly throughout the foam to revealing the indelible cross on the top of PU. In contrast, the blue GELMA spots exposed to the solution with zero papain (negative control) had remained intact throughout the whole time-course, with no observable signs of structural integrity loss (Figures 7 and 8). In these squares, the blue coloured circular spot remained, and the blue cross marked on top of the PU film had not been revealed.

### Conclusion

From these results it is clear that the cross-linked GELMA mixed with blue polystyrene microspheres remains intact when exposed to aqueous solutions in which there is no protease activity. When protease activity is introduced to the sample solution, the GELMA is readily broken down by papain (as a representative protease), so releasing the blue microspheres allowing them to disperse throughout the underlying foam layer. This change in state is indicative of protease activity and can be observed either as simple colour dispersal or as the reveal of a superimposed permanent colour-matched cross (or other symbol).

This result has also been observed with neutrophil elastase and matrix metalloprotease 9, both of which are expected to be present in infected or inflamed wound fluids, as shown in Example 5.

### Example 5: Demonstration of a product according to the invention in use based on methacrylate cross-linked gelatin

### Materials used

GelMA: modified gelatin with methacrylic anhydride. (Methacrylic anhydride sourced from Sigma-Aldrich (product number 276685). Gelatin sourced from Sigma Aldrich, Porcine Type A, 175 bloom (product number G2625))

Photo initiator "Daracure", 2-hydroxy-4'-(2hydroxyethoxy)-2-methylpropiophenone sourced from Sigma Aldrich (product number 410896)

Polybead® Polystyrene Blur Dyed Microsphere (0.5 micron diameter, 2.5% solids) sourced from Polysciences, Inc. (product number 15709)

Orion non-woven fabric (4 osy weight) sourced from Anowo Ltd.

### Method

A 0.5% w/v photo initiator (PI) solution was prepared by adding 2.5mg of Daracure powder to 500µl 1x concentration phosphate buffered saline (PBS). The mixture was heated to 60°C with periodic vortex mixing until all solids had dissolved. After the photo initiator had been completely dissolved, 50mg of GELMA powder was added to the solution, which was gently mixed to allow the GELMA to dissolve. During this dissolution process, the temperature of the GELMA/PI/PBS solution was reduced to 40°C with periodic vortex mixing. The temperature of the solution was maintained at 40°C to prevent the GELMA solidifying to a gel. Blue polystyrene microspheres were finally added at a ratio of 9 parts GELMA/PI/PBS to 1 part micro particle stock solution. The solution was mixed thoroughly to ensure a homogenous solution. The final concentrations within the mixture were: 0.45% photo initiator, 8.1% GELMA and 0.25% micro particles.

1µl aliquots of the GELMA/microsphere/photo-initiator mix were taken and deposited centrally onto 5 x 5mm squares of the Orion non-woven material. A series of repeat depositions were made. The drop was allowed to diffuse into the Orion pad, before being placed under a broad spectrum UV lamp (Dr. Honle, Germany; power c.100mW/cm²) for 15 seconds to initiate crosslinking and polymerise the GELMA. Finally the polymerised spots on the Orion squares were air-dried at ambient temperature for 3 hours.

To test the responsiveness of the assembled indicators, a series of test solutions were prepared. Matrix metalloproteinase 9 (MMP9) and human neutrophil elastase (HNE) enzymes were diluted into activation buffer with the following composition:- 50mM Tris buffer, 10mM calcium chloride dihydrate, 100mM sodium chloride, 50µM zinc chloride, 0.025% w/w Brij 35, 0.05% sodium azide in deionised water. Each enzyme was diluted in the activation buffer to give the following enzyme concentrations: 0, 0.15, 0.31, 0.62, 1.25, 2.5, 5 and 10 micrograms/ml. Two of the prepared indicator squares for each enzyme dilution were placed in a petri dish, providing a series of material squares in an 8 x 2 formation. To each pairing, 25µL of the relevant enzyme dilution was added per square. The squares were photographed before they were placed in contact with the test solutions (i.e. in a dry state) and again when initially wetted with the test solutions, and yet again when 24, 48, 120 and 144 hours at 37°C had elapsed after first contact with the test solutions.

### Results

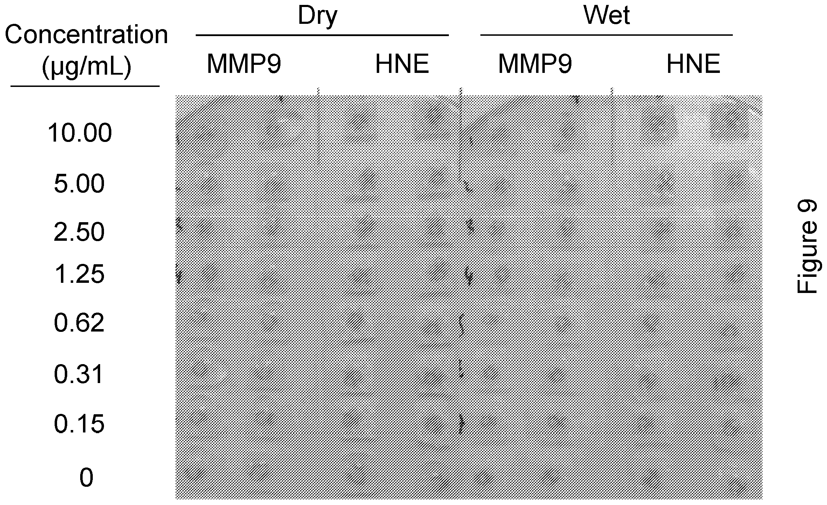

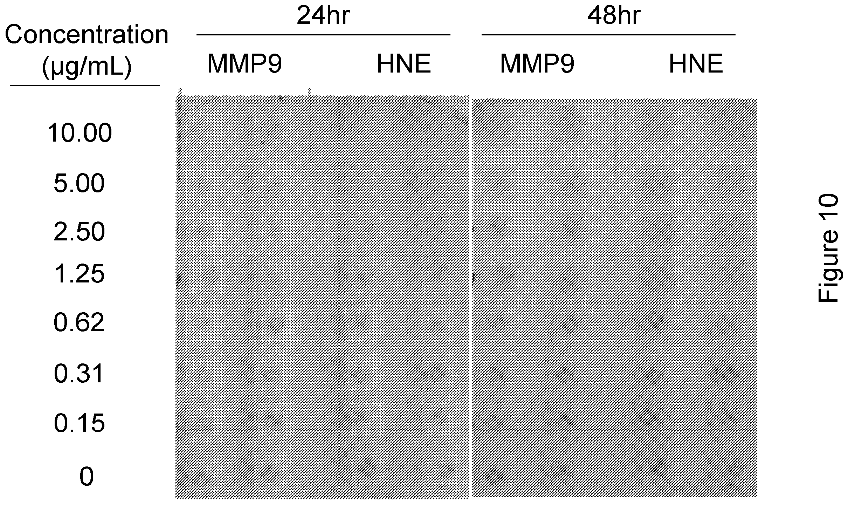

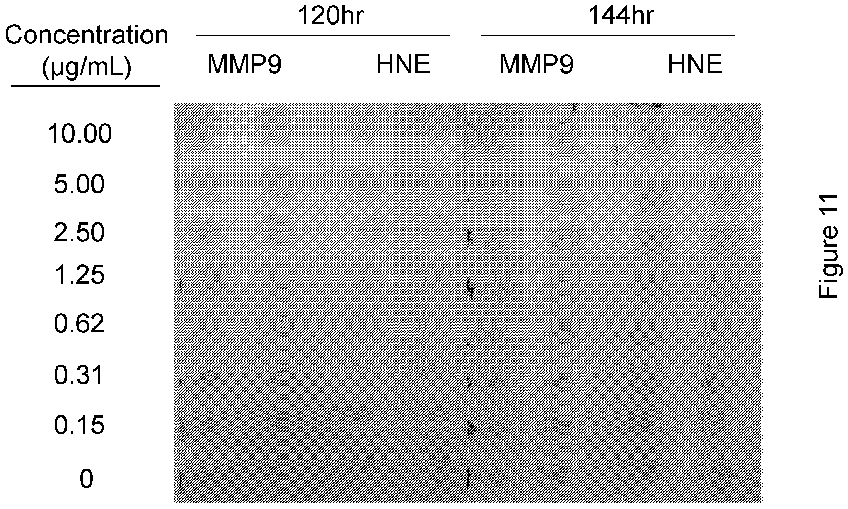

The results of the experiment are shown in Figures 9-11. In the dry state and on first wetting (i.e. time = 0hr) with any of the test solutions it can be seen that the blue GELMA spots are completely intact (Figure 9). At each of the subsequent time points, digestion of the GELMA was observed in the presence of both MMP9 and HNE (Figures 10 and 11). This is indicated by the dispersal (and attenuation) in colour throughout the Orion non-woven material. This is because the gelatin component of the GELMA has been digested by the active protease enzyme, which in turn releases the entrapped and immobilised microspheres. The coloured microspheres then disperse, spreading evenly throughout the Orion non-woven material. In contrast, the blue GELMA spots exposed to the solution which did not contain MMP9 or HNE (0 µg/ml; negative control) remained intact throughout the whole time-course, with no observable signs of loss of structural integrity (Figures 9-11). The results therefore demonstrate the visible attenuation/loss of colour when the GELMA is exposed to active MMP9 and HNE protease activity. At 24 hours, the higher concentration of both enzymes digested the GELMA and lost the coloured spot (present at time point = 0hr) with the coloured microspheres dispersing throughout the Orion non-woven material. As the incubation time increased, lower concentrations of active enzyme were able to cleave the GELMA and release the coloured microspheres resulting in colour attenuation/loss (Figures 10 and 11). This time course clearly demonstrates the GELMA is digestible by both MMP9 and HNE enzymes. The zero enzyme control spots remained intact throughout the test, confirming the crosslinked GELMA does not diffuse or dissolve at 37°C over an extended incubation period (144 hr).

### Conclusion

From these results it is clear that the cross-linked GELMA mixed with blue polystyrene microspheres remains intact when exposed to aqueous solutions in which there is no protease activity. When active protease activity is present, the GELMA is readily broken down by MMP9 and HNE (representative of biologically relevant enzymes in a wound), so releasing the blue microspheres allowing them to disperse throughout the underlying absorbent layer. This change in state is indicative of protease activity and can be observed either as simple colour dispersal or, in some embodiments, by the consequent revelation of a symbol (e.g. a cross) superimposed or underneath the initial colour spot.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all aspects and embodiments of the invention described herein are considered to be broadly applicable and combinable with any and all other consistent embodiments, including those taken from other aspects of the invention (including in isolation) as appropriate.

## Claims

1. A product for monitoring the condition of a wound comprising:
a. a sample application zone to which wound fluid is added
b. a reaction zone downstream of the sample application zone comprising protease-sensitive polymers;
c. a zone comprising coloured particles downstream of the sample application zone; and
d. a viewing zone downstream of the sample application zone, reaction zone and zone comprising coloured particles;
wherein the sample application zone transmits fluid towards the viewing zone and wherein cleavage of the polymers by protease activity present in the wound fluid results in carriage of the coloured particles with the wound fluid to the viewing zone thereby providing a visual indication of protease activity in the viewing zone, wherein:
(i) the coloured particles are entrapped within the polymers and cleavage of the polymers by protease activity present in the wound fluid results in release of the coloured particles to the viewing zone thereby providing a visual indication of protease activity in the wound fluid via the viewing zone; or
(ii) the reaction zone forms a barrier that prevents wound fluid reaching the viewing zone and wherein cleavage of the polymers by protease activity present in the wound fluid, optionally above a threshold level, disrupts the barrier and results in carriage of the coloured particles to the viewing zone thereby providing a visual indication of protease activity in the wound fluid via the viewing zone.

2. The product of claim 1 wherein:
a. the polymers are cross-linked, optionally
wherein the cross-links:
(i) comprise methacrylate or derivatives thereof; or
(ii) are derived from glutaraldehyde or derivatives thereof; and/or
b. the zone comprising coloured particles of claim 1 ii) is downstream of the reaction zone; and/or
c. the product comprises a visual symbol that, prior to exposure to wound fluid and in the absence of protease activity, is masked by the coloured particles and wherein the visual indication of protease activity in the wound fluid comprises revelation of the visual symbol, optionally wherein this defines a second viewing zone positioned above the zone comprising coloured particles prior to exposure to wound fluid and in the absence of protease activity; and/or
d. the viewing zone comprises capture molecules to capture coloured particles in the viewing zone, optionally
wherein the capture molecules comprise antibodies that bind specifically to the coloured particles; and/or
e. the product comprises a barrier at the downstream end of the viewing zone downstream of the sample application zone, reaction zone and zone comprising coloured particles so that coloured particles accumulate in the said viewing zone; and/or
f. the sample application zone and reaction zone at least partially overlap; and/or
g. the coloured particles comprise polystyrene microparticles.

3. A method of monitoring the condition of a wound comprising:
a. applying a sample of wound fluid to the sample application zone of a product as claimed in claim 1 or 2; and
b. detecting the visual indication of protease activity provided by the coloured particles in the viewing zone.

4. The method of claim 3 wherein:
a. the measured coloured particles in the viewing zone provide a quantitation of the protease activity in the wound fluid; and/or
b. the product further comprises a second viewing zone and loss of coloured particles in the second zone is compared with gain in coloured particles in the first zone.

5. A product for monitoring the condition of a wound comprising a matrix that absorbs wound fluid, the matrix comprising:
a. cross-linked and protease-sensitive polymers forming a reaction zone on/in the matrix; and
b. coloured particles;
wherein the arrangement of the polymers and coloured particles is such that cleavage of the polymers by protease activity present in the wound fluid results in transport of the coloured particles along/through the matrix to provide a visual indication of protease activity in the wound fluid;
wherein the matrix comprises a viewing zone that, prior to exposure to wound fluid and in the absence of protease activity, does not contain coloured particles and wherein cleavage of the polymers by protease activity present in the wound fluid results in release of the coloured particles along/through the matrix providing a visual indication of protease activity in the viewing zone, wherein:
(i) the coloured particles are entrapped within the polymers and cleavage of the polymers by protease activity present in the wound fluid results in release of the coloured particles along/through the matrix to provide a visual indication of protease activity in the wound fluid; or
(ii) the cross-linked and protease-sensitive polymers form a barrier that, in the absence of protease activity, optionally above a threshold level, in the wound fluid, prevents the wound fluid from coming into contact with the coloured particles and wherein cleavage of the polymers by protease activity present in the wound fluid, optionally above a threshold level, disrupts the barrier and results in flow of the coloured particles along/through the matrix to provide a visual indication of protease activity in the wound fluid in the viewing zone.

6. The product of claim 5 wherein the visual indication comprises dispersal of the coloured particles.

7. The product of claim 5(i) or 6 wherein the matrix comprises a visual symbol that, prior to exposure to wound fluid and in the absence of protease activity, is masked by the coloured particles entrapped within the polymers and wherein the visual indication of protease activity in the wound fluid comprises revelation of the visual symbol as the polymers are cleaved and the coloured particles released.

8. The product of any one of claims 5-7 wherein:
a. the matrix visible in the viewing zone comprises capture molecules to capture coloured particles; or
b. the matrix comprises a barrier aligned with the (downstream) end of the viewing zone so that coloured particles accumulate in the viewing zone; and/or
c. the coloured particles comprise polystyrene microparticles; and/or
d. the cross-links:
(i) comprise methacrylate or derivatives thereof; or
(ii) are derived from glutaraldehyde or derivatives thereof.

9. The product of any one of claims 5(ii) to 8 wherein the matrix comprises a visual symbol that, prior to exposure to wound fluid and in the absence of protease activity, is masked by the coloured particles and wherein the visual indication of protease activity in the wound fluid comprises revelation of the visual symbol as the polymers are cleaved, the barrier disrupted and the coloured particles flow along/through the matrix.

10. The product of any one of claims 1 to 2 or 5-9 wherein:
a. the polymers comprise collagen polymers; and/or
b. the polymers comprise gelatin polymers; and/or
c. the protease is a serine protease, cysteine protease, aspartic protease, threonine protease and/or glutamic protease.

11. A method of monitoring the condition of a wound comprising:
a. applying a sample of wound fluid to a product as claimed in any one of claims 5 to 10; and
b. detecting the visual indication of protease activity provided by the coloured particles.

12. The method of claim 11 wherein the coloured particles are measured in a region of the matrix to provide a quantitation of the protease activity in the wound fluid, optionally wherein:
a. the region comprises a viewing zone; and/or
b. there are two viewing zones and loss of coloured particles in one zone is compared with gain in coloured particles in a second zone.

## Patentansprüche

1. Produkt zur Überwachung des Zustands einer Wunde, umfassend:
a. eine Probeapplikationszone, der Wundfluid zugefügt wird
b. eine der Probenapplikationszone nachgeschaltete Reaktionszone, die Protease-empfindliche Polymere umfasst;
c. eine Zone, die Farbpartikel der Probenapplikationszone nachgeschaltet umfasst; und
d. eine Sichtzone, die der Probenapplikationszone, der Reaktionszone und der Farbpartikel umfassenden Zone nachgeschaltet ist;
wobei die Probenapplikationszone Fluid in Richtung der Sichtzone überträgt und wobei die Spaltung der Polymere durch in dem Wundfluid vorhandene Proteaseaktivität zum Weitertransport der der Farbpartikel mit dem Wundfluid zur Sichtzone führt, wodurch eine visuelle Anzeige von Proteaseaktivität in der Sichtzone bereitgestellt wird, wobei
(i) die Farbpartikel in den Polymeren eingeschlossen sind und die Spaltung der Polymere durch in dem Wundfluid vorhandene Proteaseaktivität zur Freisetzung der Farbpartikel in die Sichtzone führt, wodurch eine visuelle Anzeige von Proteaseaktivität in dem Wundfluid über die Sichtzone bereitgestellt wird; oder
(ii) die Reaktionszone eine Barriere bildet, die verhindert, dass Wundfluid die Sichtzone erreicht, und wobei die Spaltung der Polymere durch in dem Wundfluid vorhandene Proteaseaktivität, gegebenenfalls oberhalb eines Schwellenniveaus, die Barriere unterbricht und zum Weitertransport der Farbpartikel zur Sichtzone führt, wodurch eine visuelle Anzeige von Proteaseaktivität in dem Wundfluid über die Sichtzone bereitgestellt wird.

2. Produkt nach Anspruch 1, wobei:
a. die Polymere vernetzt sind, wobei die Vernetzungen gegebenenfalls
(i) Methacrylat oder Derivate davon umfassen; oder
(ii) von Glutaraldehyd oder Derivaten davon abgeleitet sind; und/oder
b. die Zone, die Farbpartikel nach Anspruch 1ii) umfasst, der Reaktionszone nachgeschaltet ist; und/oder
c. das Produkt ein visuelles Symbol umfasst, das, vor Exposition gegenüber Wundfluid und in Abwesenheit von Proteaseaktivität, durch die Farbpartikel maskiert ist, und wobei die visuelle Anzeige von Proteaseaktivität in dem Wundfluid die Enthüllung des visuellen Symbols umfasst, wobei dies gegebenenfalls eine zweite Sichtzone, die über der Zone, die Farbpartikel umfasst, positioniert ist, vor der Exposition gegenüber Wundfluid und in Abwesenheit von Proteaseaktivität definiert; und/oder
d. die Sichtzone Fängermoleküle enthält, um Farbpartikel in der Sichtzone einzufangen, gegebenenfalls
wobei die Fängermoleküle Antikörper umfassen, die spezifisch an die Farbpartikel binden; und/oder
e. das Produkt eine Barriere am nachgeschalteten Ende der Sichtzone der Probenapplikationszone, Reaktionszone und Zone, die Farbpartikel nachgeschaltet umfasst, so dass sich die Farbpartikel in der Sichtzone ansammeln; und/oder
f. die Probenapplikationszone und die Reaktionszone zumindest teilweise überlappen; und/oder
g. die Farbpartikel Polystyrol-Mikropartikel umfassen.

3. Verfahren zur Überwachung des Zustands einer Wunde, umfassend:
a. Aufbringen einer Probe von Wundfluid auf die Probenapplikationszone eines Produkts nach Anspruch 1 oder 2; und
b. Erkennung der visuellen Anzeige von Protease-Aktivität, die durch die Farbpartikel in der Sichtzone bereitgestellt wird.

4. Verfahren nach Anspruch 3, wobei:
a. die gemessenen Farbpartikel in der Sichtzone eine Quantifizierung der Proteaseaktivität in dem Wundfluid bereitstellen; und/oder
b. das Produkt ferner eine zweite Sichtzone umfasst, und der Verlust an Farbpartikeln in der zweiten Zone mit dem Gewinn an Farbpartikeln in der ersten Zone verglichen wird.

5. Produkt zur Überwachung des Zustands einer Wunde, das eine Matrix umfasst, die Wundfluid absorbiert, wobei die Matrix umfasst:
a. vernetzte und Protease-empfindliche Polymere, die eine Reaktionszone auf/in der Matrix bilden; und
b. Farbpartikel;
wobei die Anordnung der Polymere und Farbpartikel derart ist, dass die Spaltung der Polymere durch in dem Wundfluid vorhandene Proteaseaktivität zu einem Transport der Farbpartikel entlang/durch die Matrix führt, um eine visuelle Anzeige von Proteaseaktivität in dem Wundfluid bereitzustellen;
wobei die Matrix eine Sichtzone umfasst, die, vor der Exposition gegenüber Wundfluid und in Abwesenheit von Proteaseaktivität, keine Farbpartikel enthält, und wobei die Spaltung der Polymere durch in dem Wundfluid vorhandene Proteaseaktivität zur Freisetzung der Farbpartikel längs/durch die Matrix hindurch führt, was eine visuelle Anzeige von Proteaseaktivität in der Sichtzone bereitstellt, wobei
(i) die Farbpartikel in den Polymeren eingeschlossen sind und Spaltung der Polymere durch in dem Wundfluid vorhandene Proteaseaktivität zur Freisetzung der Farbpartikel längs/durch die Matrix hindurch führt, um eine visuelle Anzeige der Proteaseaktivität in dem Wundfluid bereitzustellen; oder
(ii) die vernetzten und Protease-empfindlichen Polymere eine Barriere bilden, die, in Abwesenheit von Proteaseaktivität, gegebenenfalls über einem Schwellenniveau, in dem Wundfluid verhindert, dass das Wundfluid mit den Farbpartikeln in Kontakt kommt, und wobei die Spaltung der Polymere durch in dem Wundfluid vorhandene Proteaseaktivität, gegebenenfalls über einem Schwellenniveau, die Barriere unterbricht und zum Fließen der Farbpartikel längs/durch die Matrix hindurch führt, um eine visuelle Anzeige von Proteaseaktivität in dem Wundfluid in der Sichtzone bereitzustellen.

6. Produkt nach Anspruch 5, wobei die visuelle Anzeige die Dispersion der Farbpartikel umfasst.

7. Produkt nach Anspruch 5(i) oder 6, wobei die Matrix ein visuelles Symbol umfasst, das, vor der Exposition gegenüber Wundfluid und in Abwesenheit von Proteaseaktivität, durch die in den Polymeren eingeschlossenen Farbpartikel maskiert ist, und wobei die visuelle Anzeige von Proteaseaktivität in dem Wundfluid die Enthüllung des visuellen Symbols umfasst, da die Polymere gespalten und die Farbpartikel freigesetzt werden.

8. Produkt nach einem der Ansprüche 5-7, wobei:
a. die in der Sichtzone sichtbare Matrix Fängermoleküle zum Einfangen von Farbpartikeln umfasst; oder
b. die Matrix eine Barriere umfasst, die mit dem (nachgeschalteteten) Ende der Sichtzone ausgerichtet ist, so dass sich Farbpartikel in der Sichtzone ansammeln; und/oder
c. die Farbpartikel Polystyrol-Mikropartikel umfassen; und/oder
d. die Vernetzungen:
(i) Methacrylat oder Derivate davon umfassen; oder
(ii) von Glutaraldehyd oder Derivaten davon abgeleitet sind.

9. Produkt nach einem der Ansprüche 5(ii) bis 8, wobei die Matrix ein visuelles Symbol umfasst, das vor der Exposition gegenüber Wundfluid und in Abwesenheit von Proteaseaktivität durch die Farbpartikel maskiert ist, und wobei die visuelle Anzeige von Proteaseaktivität in dem Wundfluid die Enthüllung des visuellen Symbols umfasst, da die Polymere gespalten werden, die Barriere unterbrochen wird und die Farbpartikel längs/durch die Matrix hindurch fließen.

10. Produkt nach einem der Ansprüche 1 bis 2 oder 5-9, wobei:
a. die Polymere Kollagenpolymere umfassen; und/oder
b. die Polymere Gelatinepolymere umfassen; und/oder
c. die Protease eine Serinprotease, Cysteinprotease, Asparaginsäureprotease, Threoninprotease und/oder Glutaminprotease ist.

11. Verfahren zur Überwachung des Zustands einer Wunde, umfassend:
a. Aufbringen einer Probe von Wundfluid auf ein Produkt nach einem der Ansprüche 5 bis 10; und
b. Nachweis der visuellen Anzeige von Proteaseaktivität durch die Farbpartikel .

12. Verfahren nach Anspruch 11, wobei die Farbpartikel in einem Bereich der Matrix gemessen werden, um eine Quantifizierung der Proteaseaktivität in dem Wundfluid bereitzustellen, wobei gegebenenfalls:
a. der Bereich eine Sichtzone umfasst; und/oder
b. zwei Sichtzonen vorhanden sind und der Verlust an Farbpartikeln in einer Zone mit dem Gewinn an Farbpartikeln in einer zweiten Zone verglichen wird.

## Revendications

1. Produit pour surveiller l'état d'une plaie comprenant :
a. une zone d'application d'échantillon à laquelle un fluide de plaie est ajouté
b. une zone de réaction en aval de la zone d'application d'échantillon comprenant des polymères sensibles aux protéases ;
c. une zone comprenant des particules colorées en aval de la zone d'application d'échantillon ; et
d. une zone de visualisation en aval de la zone d'application d'échantillon, de la zone de réaction et de la zone comprenant des particules colorées ;
dans lequel la zone d'application d'échantillon transmet un fluide vers la zone de visualisation et dans lequel le clivage des polymères par l'activité de protéase présente dans le fluide de plaie entraîne le transport des particules colorées avec le fluide de plaie vers la zone de visualisation, fournissant ainsi une indication visuelle de l'activité de protéase dans la zone de visualisation, où :
(i) les particules colorées sont piégées dans les polymères et le clivage des polymères par l'activité de protéase présente dans le fluide de plaie entraîne la libération des particules colorées à la zone de visualisation, fournissant ainsi une indication visuelle de l'activité de protéase dans le fluide de plaie par l'intermédiaire de la zone de visualisation ; ou
(ii) la zone de réaction forme une barrière qui empêche le fluide de plaie d'atteindre la zone de visualisation et où le clivage des polymères par l'activité de protéase présente dans le fluide de plaie, facultativement au-dessus d'un niveau seuil, rompt la barrière et entraîne le transport des particules colorées vers la zone de visualisation, fournissant ainsi une indication visuelle de l'activité de protéase dans le fluide de plaie par l'intermédiaire de la zone de visualisation.

2. Produit de la revendication 1, dans lequel :
a. les polymères sont réticulés, facultativement où les réticulations :
(i) comprennent du méthacrylate ou ses dérivés ; ou
(ii) sont dérivées du glutaraldéhyde ou de ses dérivés ; et/ou
b. la zone comprenant des particules colorées de la revendication 1 ii) se trouve en aval de la zone de réaction ; et/ou
c. le produit comprend un symbole visuel qui, avant l'exposition au fluide de plaie et en l'absence d'activité de protéase, est masqué par les particules colorées et où l'indication visuelle de l'activité de protéase dans le fluide de plaie comprend la révélation du symbole visuel, facultativement où ceci définit une deuxième zone de visualisation positionnée au-dessus de la zone comprenant des particules colorées avant l'exposition au fluide de plaie et en l'absence d'activité de protéase ; et/ou
d. la zone de visualisation comprend des molécules de capture pour capturer des particules colorées dans la zone de visualisation, facultativement
où les molécules de capture comprennent des anticorps qui se lient spécifiquement aux particules colorées ; et/ou
e. le produit comprend une barrière au niveau de l'extrémité aval de la zone de visualisation en aval de la zone d'application d'échantillon, de la zone de réaction et de la zone comprenant des particules colorées de sorte que les particules colorées s'accumulent dans ladite zone de visualisation ; et/ou
f. la zone d'application d'échantillon et la zone de réaction se chevauchent au moins partiellement ; et/ou
g. les particules colorées comprennent des micro-particules de polystyrène.

3. Procédé de surveillance de l'état d'une plaie comprenant les étapes consistant à :
a. appliquer un échantillon de fluide de plaie à la zone d'application d'échantillon d'un produit tel que revendiqué dans la revendication 1 ou 2 ; et
b. détecter l'indication visuelle de l'activité de protéase fournie par les particules colorées dans la zone de visualisation.

4. Procédé de la revendication 3, dans lequel :
a. les particules colorées mesurées dans la zone de visualisation fournissent une quantification de l'activité de protéase dans le fluide de plaie ; et/ou
b. le produit comprend en outre une deuxième zone de visualisation et la perte de particules colorées dans la deuxième zone est comparée au gain en particules colorées dans la première zone.

5. Produit pour surveiller l'état d'une plaie comprenant une matrice qui absorbe le fluide de plaie, la matrice comprenant :
a. des polymères réticulés et sensibles aux protéases formant une zone de réaction sur/dans la matrice ; et
b. des particules colorées ;
dans lequel l'agencement des polymères et des particules colorées est tel que le clivage des polymères par l'activité de protéase présente dans le fluide de plaie entraîne le transport des particules colorées le long de/à travers la matrice pour fournir une indication visuelle de l'activité de protéase dans le fluide de plaie ;
dans lequel la matrice comprend une zone de visualisation qui, avant l'exposition au fluide de plaie et en l'absence d'activité de protéase, ne contient pas de particules colorées et où le clivage des polymères par l'activité de protéase présente dans le fluide de plaie entraîne la libération des particules colorées le long de/à travers la matrice fournissant une indication visuelle de l'activité de protéase dans la zone de visualisation, où :
(i) les particules colorées sont piégées dans les polymères et le clivage des polymères par l'activité de protéase présente dans le fluide de plaie entraîne la libération des particules colorées le long de/à travers la matrice pour fournir une indication visuelle de l'activité de protéase dans le fluide de plaie ; ou
(ii) les polymères réticulés et sensibles aux protéases forment une barrière qui, en l'absence d'activité de protéase, facultativement au-dessus d'un niveau seuil, dans le fluide de plaie, empêche le fluide de plaie d'entrer en contact avec les particules colorées et où le clivage des polymères par l'activité de protéase présente dans le fluide de plaie, facultativement au-dessus d'un niveau seuil, rompt la barrière et entraîne l'écoulement des particules colorées le long de/à travers la matrice pour fournir une indication visuelle de l'activité de protéase dans le fluide de plaie dans la zone de visualisation .

6. Produit de la revendication 5, dans lequel l'indication visuelle comprend la dispersion des particules colorées.

7. Produit de la revendication 5(i) ou 6, dans lequel la matrice comprend un symbole visuel qui, avant l'exposition au fluide de plaie et en l'absence d'activité de protéase, est masqué par les particules colorées piégées dans les polymères et dans lequel l'indication visuelle de l'activité de protéase dans le fluide de plaie comprend la révélation du symbole visuel à mesure que les polymères sont clivés et les particules colorées sont libérées.

8. Produit de l'une quelconque des revendications 5 à 7, dans lequel :
a. la matrice visible dans la zone de visualisation comprend des molécules de capture pour capturer des particules colorées ; ou
b. la matrice comprend une barrière alignée avec l'extrémité (aval) de la zone de visualisation de sorte que les particules colorées s'accumulent dans la zone de visualisation ; et/ou
c. les particules colorées comprennent des micro-particules de polystyrène ; et/ou
d. les réticulations :
(i) comprennent du méthacrylate ou ses dérivés ; ou
(ii) sont dérivées du glutaraldéhyde ou de ses dérivés.

9. Produit de l'une quelconque des revendications 5(ii) à 8, dans lequel la matrice comprend un symbole visuel qui, avant l'exposition au fluide de plaie et en l'absence d'activité de protéase, est masqué par les particules colorées et dans lequel l'indication visuelle de l'activité de protéase dans le fluide de plaie comprend la révélation du symbole visuel à mesure que les polymères sont clivés, la barrière est rompue et les particules colorées s'écoulent le long de/à travers la matrice.

10. Produit de l'une quelconque des revendications 1 et 2 ou 5 à 9, dans lequel :
a. les polymères comprennent des polymères de collagène ; et/ou
b. les polymères comprennent des polymères de gélatine ; et/ou
c. la protéase est une sérine protéase, une cystéine protéase, une protéase aspartique, une thréonine protéase et/ou une protéase glutamique.

11. Procédé de surveillance de l'état d'une plaie comprenant les étapes consistant à :
a. appliquer un échantillon de fluide de plaie à un produit tel que revendiqué dans l'une quelconque des revendications 5 à 10 ; et
b. détecter l'indication visuelle de l'activité de protéase fournie par les particules colorées.

12. Procédé de la revendication 11, dans lequel les particules colorées sont mesurées dans une région de la matrice pour fournir une quantification de l'activité de protéase dans le fluide de plaie, facultativement dans lequel :
a. la région comprend une zone de visualisation ; et/ou
b. il y a deux zones de visualisation et la perte de particules colorées dans une zone est comparée au gain en particules colorées dans une deuxième zone.
